# EUROPEAN PATENT APPLICATION

(11) **EP 4 595 915 A1**
(43) Date of publication of application: **06.08.2025**
(21) Application number: 25154656.0
(22) Date of filing: 29.01.2025
(51) Int. Cl.: A61B 34/10, A61B 17/17, A61B 34/20, A61B 34/30, A61B 90/00

(54) **COMPUTER ASSISTED PELVIC SURGERY NAVIGATION**

(30) Priority: 01.02.2024 US 202418430077
(71) Applicant: Globus Medical, Inc., Audubon, PA 19403 (US)
(72) Inventor: CAMERON, Hayden, Philadelphia, PA 19129 (US); DUCH, Loris, 1053 VAUD (CH)
(74) Representative: Prendin, Marco

(57) **Abstract**

A system for computer assisted navigation during surgery includes computer platform that operates to identify a set of locations at which a navigated instrument is palpitating a landmark defined on a surface of a pelvic bone of a patient. Further operations determine a center of rotation for a pelvic acetabulum of the patient based on the identified set of locations at which the navigated instrument is palpitating the landmark. Operations determine an orientation of an anterior pelvic plane (APP) and/or a functional pelvic plane (FPP) of the patient based on the identified set of locations at which the navigated instrument is palpitating the landmark and based on the determined center of rotation for the pelvic acetabulum.

## Description

### FIELD

The present disclosure relates to medical devices and systems, and more particularly, surgical navigation systems for pelvic surgery.

### BACKGROUND

Computer assisted surgery navigation systems have become a well-established technique in operating rooms for providing surgeons with computerized visualization of how a surgical instrument or other device that is posed relative to a patient correlates to a pose relative to medical images of the patient's anatomy, and how those poses correlate to a pre-operative surgical plan. Camera tracking systems for computer assisted surgery navigation typically use a set of tracking cameras to track pose of a reference element on the surgical instrument, which is being positioned by a surgeon during surgery, relative to a patient reference element (also "dynamic reference base" (DRB)) affixed to a patient. A computer model of a real instrument is associated with a reference element, so that the computer model can be overlaid on registered images of patient's anatomy. The camera tracking system uses the relative poses of the reference elements to determine how the real instrument is posed relative to a patient and to determine how the computer model of the real instrument is to be correspondingly posed as on overlay on the medical images. The surgeon can thereby use real-time visual feedback of the relative poses to navigate the surgical instrument during a surgical procedure on the patient.

A robotic system for knee replacement can be used which has a serial arm on which a passive structure guiding the saw blade is mounted. For example, a sagittal saw can be attached to the end of the passive structure to guide the cutting plane. The system can enable a surgeon to hold the sagittal saw and cut bones while watching on the navigation system (e.g., stand-alone displays or Augmented Reality (AR) headset) various types of relevant feedback and information associated with a defined plan for and/or progress of the surgical procedure.

The serial arm can moved through computer guided control to a suitable position for the surgery, e.g., pursuant to the surgeon's request which may be provided via a foot pedal, touchscreen, AR interaction, etc. The passive structure allows the surgeon to precisely remove bone in the cutting plane. Bone removal progression can be measured through camera tracking of fiducials of a reference element attached to the bones and to the sagittal saw.

Various workflows can be available for use with the system. Some workflows require preoperative scans or images of the patient (e.g., x-ray, Computerized Tomography (CT)). On the other hand, an imageless workflow does not require any pre-operative images. To obtain intra-operative information about the patient anatomy, the surgeon measures key parameters of the bone using a camera tracking system and appropriate tracked instrument to capture points on patient anatomy. Later, this information is used to plan the implant position and orientation with respect to patient anatomy and navigate the robot and surgical instruments during the surgical procedure.

Some workflows include having the surgeon rigidly attach a reference element to one or more bones, where the reference element includes fiducials which are detected by tracking cameras for computer assisted navigation. The reference elements allow tracking of bone position by the navigation system. The reference elements can be positioned on the bone in the following way: attached with fixation structures (e.g., screw pins, "crocodile" jaws) on a bone (e.g., pelvis or femur and tibia depending on surgical procedure being performed) and oriented so as they can be seen by the tracking cameras of the navigation system. The reference elements positions and orientations must stay rigidly fixed with respect to the bone.

Another step of various workflows is to register the patient in tracking space of the navigation system. Patient registration can include matching the patient anatomy with numeric representation of the corresponding bone, usually a 3D model of the bone. The bone representation may be either constructed from a set of CT images (CT workflow) or based on a generic bone model (Imageless workflow).

Although current surgical procedures offer sophisticated computer assisted navigation once bone landmarks of a patient have been properly registered for tracking, current procedures for registration should be improved to be more automated and result in more accurate assisted navigation during surgery.

### SUMMARY

According to the invention, a system for computer assisted navigation during surgery is provided, the system having the features of claim 1. Further advantageous aspects of the invention are set forth in the dependent claims.

Some embodiments of the present disclosure are directed to a system for computer assisted navigation during surgery. The system includes a computer platform that operates to identify a set of locations at which a navigated instrument is contacting a landmark defined on a surface of a pelvic bone, femur or both of a patient. Further operations determine a center of rotation for a pelvic acetabulum of the patient based on the identified set of locations at which the navigated instrument is palpating the landmark. Operations determine an orientation of an anterior pelvic plane (APP) and/or a functional pelvic plane (FPP) of the patient based on the identified set of locations at which the navigated instrument is contacting the landmark and based on the determined center of rotation for the pelvic acetabulum.

Some other corresponding embodiments of the present disclosure are directed to a computer program product comprising a non-transitory computer readable medium storing instructions executable by at least one processor to perform operations for computer assisted navigation during surgery. The operations identify a set of locations at which a navigated instrument is palpating a landmark defined on a surface of a pelvic bone of a patient. Further operations determine a center of rotation for a pelvic acetabulum of the patient based on the identified set of locations at which the navigated instrument is palpating the landmark. Operations determine an orientation of an anterior pelvic plane (APP) and/or a functional pelvic plane (FPP) of the patient based on the identified set of locations at which the navigated instrument is palpating the landmark and based on the determined center of rotation for the pelvic acetabulum.

Other system for computer assisted navigation during surgery, computer program products, and related methods for computer assisted navigation during surgery according to embodiments of the inventive subject matter will be or become apparent to one with skill in the art upon review of the following drawings and detailed description. It is intended that all such additional systems, computer program products, and methods be included within this description, be within the scope of the present inventive subject matter, and be protected by the accompanying claims. Moreover, it is intended that all embodiments disclosed herein can be implemented separately or combined in any way and/or combination.

According to some embodiments of the present invention, the computer assisted navigation during surgery may comprise a tracking system configured to track the navigated instrument.

According to some embodiments of the present invention, the computer assisted navigation during surgery may comprise the navigated instrument.

### DESCRIPTION OF THE DRAWINGS

Aspects of the present disclosure are illustrated by way of example and are not limited by the accompanying drawings. In the drawings:
FIG. 1 is an overhead view of a surgical system arranged during a surgical procedure in a surgical room which includes a camera tracking system for computer assisted navigation during surgery and which may further include a surgical robot for robotic assistance according to some embodiments;
FIG. 2 illustrates the camera tracking system and the surgical robot positioned relative to a patient according to some embodiments;
FIG. 3 further illustrates the camera tracking system and the surgical robot configured according to some embodiments;
FIG. 4 illustrates a block diagram of a surgical system that includes an extended reality headset, a computer platform, imaging devices, and a surgical robot which are configured to operate according to some embodiments;
FIG. 5 illustrates the ball tip stylus configured in accordance with some embodiments of the present disclosure;
FIG. 6 illustrates a user interface displayed to guide a user through registration of condylar surfaces by palpation using the ball tip stylus in accordance with some embodiments of the present disclosure;
FIG. 7 illustrates a schematic view of operations for defining and then translating an offset-acquired surface of a bone toward the surface of the bone along local normal vectors based on a radius of the ball to define an acquired surface of the bone in accordance with some embodiments of the present disclosure;
FIG. 8 illustrates a flowchart of an imageless workflow during an intra-operative portion of a total hip arthroplasty (THA) surgery, in accordance with some embodiments of the present disclosure;
FIG. 9 illustrates a flowchart of a patient preparation process before registration, in accordance with some embodiments of the present disclosure;
FIG. 10A illustrates a radiographic inclination angle measured in the coronal plane of the patient, in accordance with some embodiments of the present disclosure;
FIG. 10B illustrates a radiographic version angle measured relative to the coronal plane of the patient, in accordance with some embodiments of the present disclosure;
FIG. 11 illustrates different views of landmarks and axes for registration of a functional pelvic plane (FPP) and an anterior pelvic plane (APP) of a patient, in accordance with some embodiments of the present disclosure;
FIG. 12 illustrates a flowchart for registration of the APP and FPP of the patient, in accordance with some embodiments of the present disclosure;
FIG. 13A-C illustrates examples of the registration of the APP and/or FPP using a tracking marker plane of a navigated instrument, in accordance with some embodiments of the present disclosure;
FIG. 14 illustrates examples of the registration of the APP and/or FPP using an axis of the navigated instrument and a tracking camera which includes an inertial measurement unit (IMU), in accordance with some embodiments of the present disclosure;
FIG. 15 illustrates a flowchart of operations for measuring a leg length and offset of the patient, in accordance with some embodiments of the present disclosure;
FIG. 16 illustrates a flowchart for registration of a pelvic acetabulum of the patient, in accordance with some embodiments of the present disclosure;
FIG. 17 illustrates operations painting the acetabular cavity with the navigated instrument, in accordance with some embodiments of the present disclosure;
FIG. 18 illustrates a user interface for a planning view which displays a graphical representation of generic bone with extracted (e.g., registered) landmarks (shown as bright points), and a graphical representation of what surfaces have been acquired through palpation operations;
FIG. 19 illustrates a flowchart of operation that can be performed by a computer platform of a system for computer assisted navigation during surgery, in accordance with some embodiments of the present disclosure;
FIG. 20 illustrates a flowchart of additional or alternative operations performed by the computer platform of the system for computer assisted navigation during surgery of the patient, in accordance with some embodiments of the present disclosure;
FIG. 21 illustrates a schematic view of operations for acquiring femoral and/or tibial features in accordance with some embodiments of the present disclosure; and
FIG. 22 illustrates a schematic view of structure and operations that may be used for the second operational approach for acquiring femoral and/or tibial features.

### DETAILED DESCRIPTION

It is to be understood that the present disclosure is not limited in its application to the details of construction and the arrangement of components set forth in the description herein or illustrated in the drawings. The teachings of the present disclosure may be used and practiced in other embodiments and practiced or carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Unless specified or limited otherwise, the terms "mounted," "connected," "attached", "supported," and "coupled" and variations thereof are used broadly and encompass both direct and indirect mountings, connections, attachments, supports, and couplings. Further, "connected" and "coupled" are not restricted to physical or mechanical connections or couplings.

The following discussion is presented to enable a person skilled in the art to make and use embodiments of the present disclosure. Various modifications to the illustrated embodiments will be readily apparent to those skilled in the art, and the principles herein can be applied to other embodiments and applications without departing from embodiments of the present disclosure. Thus, the embodiments are not intended to be limited to embodiments shown, but are to be accorded the widest scope consistent with the principles and features disclosed herein. The following detailed description is to be read with reference to the figures, in which like elements in different figures have like reference numerals. The figures, which are not necessarily to scale, depict selected embodiments and are not intended to limit the scope of the embodiments. Skilled artisans will recognize the examples provided herein have many useful alternatives and fall within the scope of the embodiments.

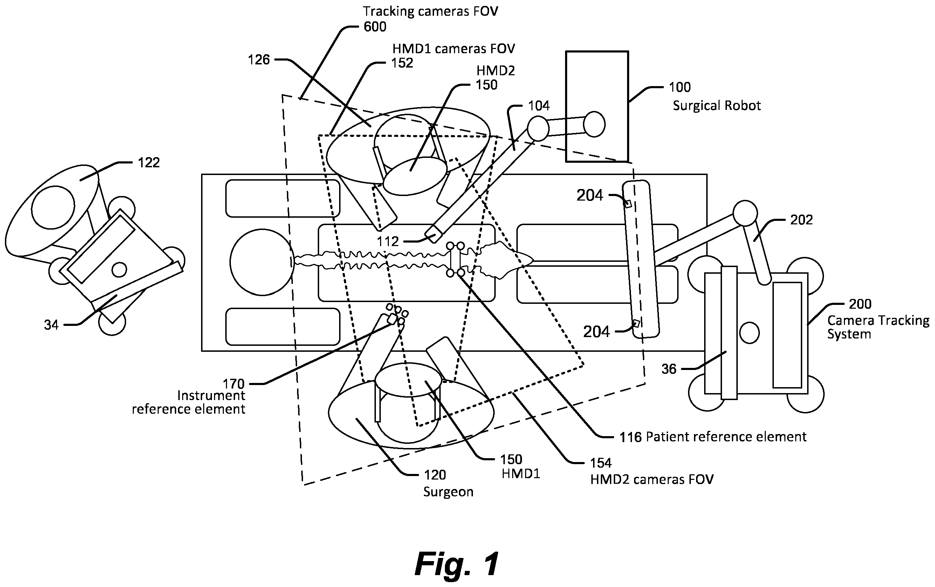

A first set of embodiments of the present disclosure are directed to a system for computer assisted navigation during surgery which operates with a navigated ball tip stylus used to contact the surfaces of bones as the stylus travels across the bones. For example, the bones can be continuously contacted by a process known as "surface painting" which refers to the user touching, e.g., tapping, the ball to individual surface locations on the bone, preferably in a zig zag fashion, and/or refer to the user touching and then dragging the ball along, preferably in a zig zag fashion, while maintaining contact with the bone surface while the stylus is tracked to enable definition of an acquired surface of the bone, in accordance with various embodiments disclosed herein. The bones can also be continuously contacted by palpation, i.e., repeatedly lifting and contacting individual surface locations while the navigated ball tip stylus travels, preferably in a zig zag fashion, over the bones. For purposes of the present application, the phrase "painting", "surface painting" and palpating will be used interchangeably to mean contact either by continuous contact or intermittent contact of the bone. Before describing these embodiments is detail, various components of a system that may be used with and/or for performing embodiments are described with reference to FIGS. 1-4.

A second set of embodiments of the present disclosure, that can be used in addition to, conjunction with, or as an alternative to the first set of embodiments, is directed to a system for computer assisted navigation during surgery which includes a computer platform that is operative to identify a set of locations at which a navigated instrument is palpating a landmark defined on a surface of a pelvic bone of a patient. The computer platform is further operative to determine a center of rotation for a pelvic acetabulum based on the identified set of locations at which the navigated instrument is palpating the landmark, and determine an orientation of an anterior pelvic plane (APP) and/or a functional pelvic plane (FPP) based on the identified set of locations at which the navigated instrument is palpating the landmark and based on the determined center of rotation for the pelvic acetabulum.

The first set of embodiments is discussed below.

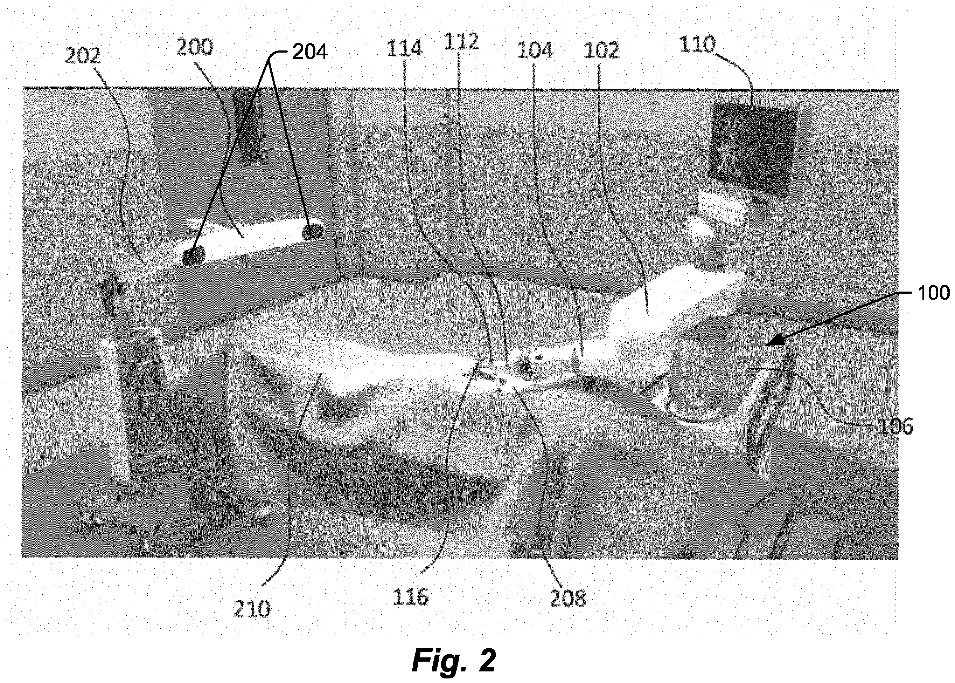

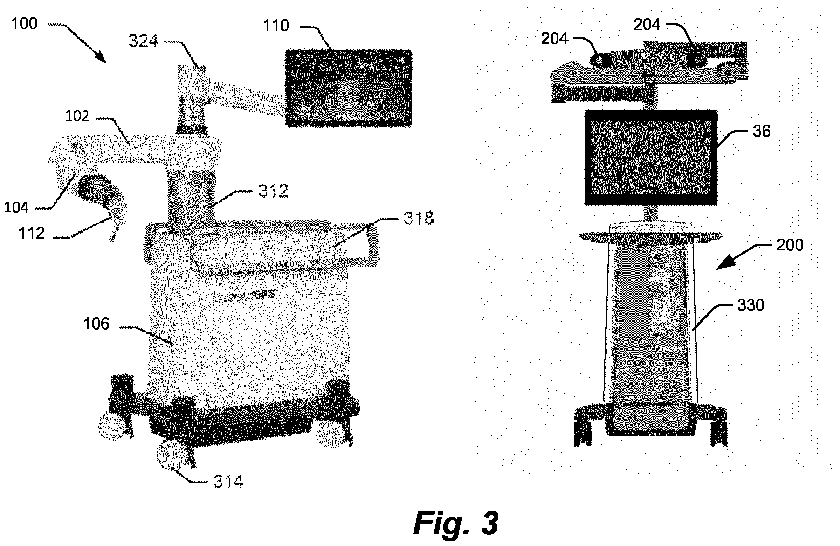

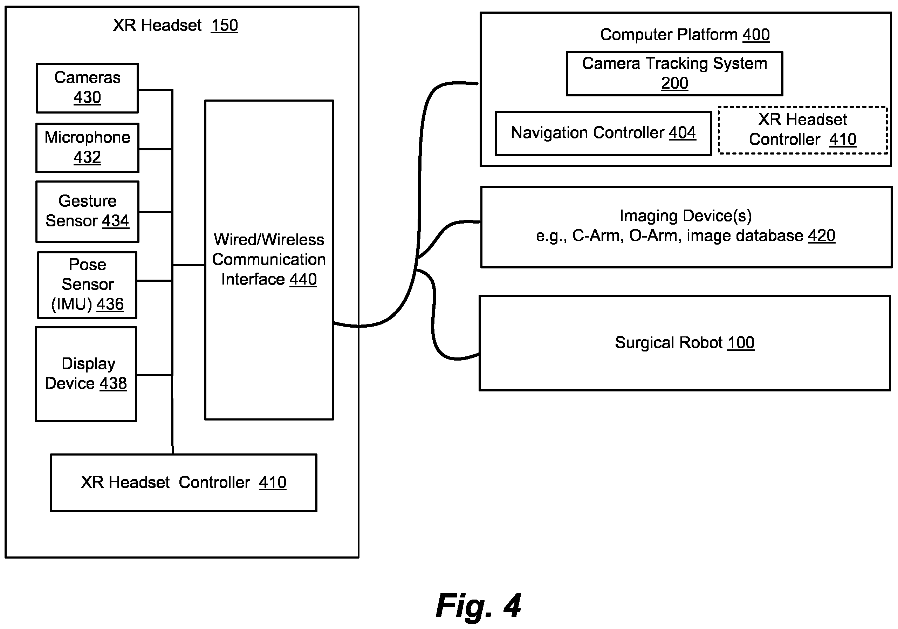

FIG. 1 is an overhead view of a surgical system arranged during a surgical procedure in a surgical room. The system includes a camera tracking system 200 for computer assisted navigation during surgery and may further include a surgical robot 100 for robotic assistance according to some embodiments. FIG. 2 illustrates the camera tracking system 200 and the surgical robot 100 positioned relative to a patient according to some embodiments. FIG. 3 further illustrates the camera tracking system 200 and the surgical robot 100 configured according to some embodiments. FIG. 4 illustrates a block diagram of a surgical system that includes an extended reality (XR) headset 150, a computer platform 400, imaging devices 420, and the surgical robot 100 which are configured to operate according to some embodiments.

The XR headsets 150 may be configured to augment a real-world scene with computer generated XR images while worn by personnel in the operating room. The XR headsets 150 may be configured to provide an augmented reality (AR) viewing environment by displaying the computer generated XR images on a see-through display screen that allows light from the real-world scene to pass therethrough for combined viewing by the user. Alternatively, the XR headsets 150 may be configured to provide a virtual reality (VR) viewing environment by preventing or substantially preventing light from the real-world scene from being directly viewed by the user while the user is viewing the computer-generated AR images on a display screen. The XR headsets 150 can be configured to provide both AR and VR viewing environments. Thus, the term XR headset can referred to as an AR headset or a VR headset.

Referring to FIGS. 1-4, the surgical robot 100 may include, for example, one or more robot arms 104, a display 110, an end-effector 112, for example, including a guide tube 114, and an end effector reference element which can include one or more tracking fiducials. A patient reference element 116 (DRB) has a plurality of tracking fiducials and is secured directly to the patient 210 (e.g., to a bone of the patient such as pelvis, femur or tibia). A reference element 170 is attached or formed on an instrument, surgical tool, surgical implant device, etc.

The camera tracking system 200 includes tracking cameras 204 which may be spaced apart stereo cameras configured with partially overlapping field-of-views. The camera tracking system 200 can have any suitable configuration of arm(s) 202 to move, orient, and support the tracking cameras 204 in a desired location, and may contain at least one processor operable to track location of an individual fiducial and pose of an array of fiducials of a reference element.

As used herein, the term "pose" refers to the location (e.g., along 3 orthogonal axes) and/or the rotation angle (e.g., about the 3 orthogonal axes) of fiducials (e.g., DRB) relative to another fiducial (e.g., surveillance fiducial) and/or to a defined coordinate system (e.g., camera coordinate system, navigation coordinate system, etc.). A pose may therefore be defined based on only the multidimensional location of the fiducials relative to another fiducial and/or relative to the defined coordinate system, based on only the multidimensional rotational angles of the fiducials relative to the other fiducial and/or to the defined coordinate system, or based on a combination of the multidimensional location and the multidimensional rotational angles. The term "pose" therefore is used to refer to location, rotational angle, or combination thereof.

The tracking cameras 204 may include, e.g., infrared cameras (e.g., bifocal or stereophotogrammetric cameras), operable to identify, for example, active and passive tracking fiducials for single fiducials (e.g., surveillance fiducial) and reference elements which can be formed on or attached to the patient 210 (e.g., patient reference element, DRB, etc.), end effector 112 (e.g., end effector reference element), XR headset(s) 150 worn by a surgeon 120 and/or a surgical assistant 126, etc. in a given measurement volume of a camera coordinate system while viewable from the perspective of the tracking cameras 204. The tracking cameras 204 may scan the given measurement volume and detect light that is emitted or reflected from the fiducials in order to identify and determine locations of individual fiducials and poses of the reference elements in three-dimensions. For example, active reference elements may include infrared-emitting fiducials that are activated by an electrical signal (e.g., infrared light emitting diodes (LEDs)), and passive reference elements may include retro-reflective fiducials that reflect infrared light (e.g., they reflect incoming IR radiation into the direction of the incoming light), for example, emitted by illuminators on the tracking cameras 204 or other suitable device.

The XR headsets 150 may each include tracking cameras (e.g., spaced apart stereo cameras) that can track location of a surveillance fiducial and poses of reference elements within the XR camera headset field-of-views (FOVs) 152 and 154, respectively. Accordingly, as illustrated in FIG. 1, the location of the surveillance fiducial and the poses of reference elements on various objects can be tracked while in the FOVs 152 and 154 of the XR headsets 150 and/or a FOV 600 of the tracking cameras 204.

FIGS. 1 and 2 illustrate a potential configuration for the placement of the camera tracking system 200 and the surgical robot 100 in an operating room environment. Computer assisted navigated surgery can be provided by the camera tracking system controlling the XR headsets 150 and/or other displays 34, 36, and 110 to display surgical procedure navigation information. The surgical robot 100 is optional during computer assisted navigated surgery.

The camera tracking system 200 may operate using tracking information and other information provided by multiple XR headsets 150 such as inertial tracking information and optical tracking information (frames of tracking data). The XR headsets 150 operate to display visual information and may play-out audio information to the wearer. This information can be from local sources (e.g., the surgical robot 100 and/or other medical), imaging devices 420 (FIG. 4), and remote sources (e.g., patient medical image database), and/or other electronic equipment. The camera tracking system 200 may track fiducials in 6 degrees-of-freedom (6 DOF) relative to three axes of a 3D coordinate system and rotational angles about each axis. The XR headsets 150 may also operate to track hand poses and gestures to enable gesture-based interactions with "virtual" buttons and interfaces displayed through the XR headsets 150 and can also interpret hand or finger pointing or gesturing as various defined commands. Additionally, the XR headsets 150 may have a 1-10x magnification digital color camera sensor called a digital loupe. In some embodiments, one or more of the XR headsets 150 are minimalistic XR headsets that display local or remote information but include fewer sensors and are therefore more lightweight.

An "outside-in" machine vision navigation bar supports the tracking cameras 204 and may include a color camera. The machine vision navigation bar generally has a more stable view of the environment because it does not move as often or as quickly as the XR headsets 150 while positioned on wearers' heads. The patient reference element 116 (DRB) is generally rigidly attached to the patient with stable pitch and roll relative to gravity. This local rigid patient reference 116 can serve as a common reference for reference frames relative to other tracked elements, such as a reference element on the end effector 112, instrument reference element 170, and reference elements on the XR headsets 150.

In some embodiments, at the end of the end effector, instruments are connected to perform operations such as resection, reaming, broaching, drilling and screw placement.

When present, the surgical robot (also "robot") may be positioned near or next to patient 210. The robot 100 can be positioned at any suitable location near the patient 210 depending on the area of the patient 210 undergoing the surgical procedure. The camera tracking system 200 may be separate from the robot system 100 and positioned at the foot of patient 210. This location allows the tracking camera 200 to have a direct visual line of sight to the surgical area 208. In the configuration shown, the surgeon 120 may be positioned across from the robot 100, but is still able to manipulate the end-effector 112 and the display 110. A surgical assistant 126 may be positioned across from the surgeon 120 again with access to both the end-effector 112 and the display 110. If desired, the locations of the surgeon 120 and the assistant 126 may be reversed. An anesthesiologist 122, nurse or scrub tech can operate equipment which may be connected to display information from the camera tracking system 200 on a display 34.

With respect to the other components of the robot 100, the display 110 can be attached to the surgical robot 100 or in a remote location. End-effector 112 may be coupled to the robot arm 104 and controlled by at least one motor. In some embodiments, end-effector 112 includes a guide tube 114, which is configured to receive and orient a surgical instrument, tool, or implant used to perform a surgical procedure on the patient 210. In some other embodiments, the end-effector 112 includes a passive structure guiding a saw blade (e.g., sagittal saw) along a defined cutting plate.

As used herein, the term "end-effector" is used interchangeably with the terms "end-effectuator" and "effectuator element." The term "instrument" is used in a non-limiting manner and can be used interchangeably with "tool" and "implant" to generally refer to any type of device that can be used during a surgical procedure in accordance with embodiments disclosed herein. The more general term device can also refer to structure of the end-effector, etc. Example instruments, tools, and implants include, without limitation, drills, screwdrivers, saws, dilators, retractors, probes, implant inserters, and implant devices such as a screws, spacers, interbody fusion devices, plates, rods, etc. Although generally shown with a guide tube 114, it will be appreciated that the end-effector 112 may be replaced with any suitable instrumentation suitable for use in surgery. In some embodiments, end-effector 112 can comprise any known structure for effecting the movement of the surgical instrument in a desired manner.

The surgical robot 100 is operable to control the translation and orientation of the end-effector 112. The robot 100 may move the end-effector 112 under computer control along x-, y-, and z-axes, for example. The end-effector 112 can be configured for selective rotation about one or more of the x-, y-, and z-axis , and a Z Frame axis, such that one or more of the Euler Angles (e.g., roll, pitch, and/or yaw) associated with end-effector 112 can be selectively computer controlled. In some embodiments, selective control of the translation and orientation of end-effector 112 can permit performance of medical procedures with significantly improved accuracy compared to conventional robots that utilize, for example, a 6 DOF robot arm comprising only rotational axes. For example, the surgical robot 100 may be used to operate on patient 210, and robot arm 104 can be positioned above the body of patient 210, with end-effector 112 selectively angled relative to the z-axis toward the body of patient 210.

In some example embodiments, the XR headsets 150 can be controlled to dynamically display an updated graphical indication of the pose of the surgical instrument so that the user can be aware of the pose of the surgical instrument at all times during the procedure.

In some further embodiments, surgical robot 100 can be operable to correct the path of a surgical instrument guided by the robot arm 104 if the surgical instrument strays from the selected, preplanned trajectory. The surgical robot 100 can be operable to permit stoppage, modification, and/or manual control of the movement of end-effector 112 and/or the surgical instrument. Thus, in use, a surgeon or other user can use the surgical robot 100 as part of computer assisted navigated surgery, and has the option to stop, modify, or manually control the autonomous or semi-autonomous movement of the end-effector 112 and/or the surgical instrument.

Fiducials of reference elements can be formed on or connected to robot arms 102 and/or 104, the end-effector 112 (e.g., end-effector element 114 in FIG. 2), and/or a surgical instrument (e.g., instrument element 170) to enable tracking of poses in a defined coordinate system, e.g., such as in 6 DOF along 3 orthogonal axes and rotation about the axes. The reference elements enable each of the marked objects (e.g., the end-effector 112, the patient 210, and the surgical instruments) to be tracked by the tracking camera 200, and the tracked poses can be used to provide navigated guidance during a surgical procedure and/or used to control movement of the surgical robot 100 for guiding the end-effector 112 and/or an instrument manipulated by the end-effector 112.

Referring to FIG. 3 the surgical robot 100 may include a display 110, upper arm 102, lower arm 104, end-effector 112, vertical column 312, casters 314, a table 318, and ring 324 which uses lights to indicate statuses and other information. Cabinet 106 may house electrical components of surgical robot 100 including, but not limited to, a battery, a power distribution module, a platform interface board module, and a computer. The camera tracking system 200 may include a display 36, tracking cameras 204, arm(s) 202, a computer housed in cabinet 330, and other components.

In computer assisted navigated surgeries, perpendicular 2D scan slices, such as axial, sagittal, and/or coronal views, of patient anatomical structure are displayed to enable user visualization of the patient's anatomy alongside the relative poses of surgical instruments. An XR headset or other display can be controlled to display one or more 2D scan slices of patient anatomy along with a 3D graphical model of anatomy. The 3D graphical model may be generated from a 3D scan of the patient, e.g., by a CT scan device, and/or may be generated based on a baseline model of anatomy which isn't necessarily formed from a scan of the patient.

### Example Surgical System:

FIG. 4 illustrates a block diagram of a surgical system that includes an XR headset 150, a computer platform 400, imaging devices 420, and a surgical robot 100 which are configured to operate according to some embodiments.

The imaging devices 420 may include a C-arm imaging device, an O-arm imaging device, other imaging device, and/or a patient image database (2D and/or 3D images). The XR headset 150 provides an improved human interface for performing navigated surgical procedures. The XR headset 150 can be configured to provide functionalities, e.g., via the computer platform 400, that include without limitation any one or more of: identification of hand gesture based commands, display XR graphical objects on a display device 438 of the XR headset 150 and/or another display device. The display device 438 may include a video projector, flat panel display, etc. The user may view the XR graphical objects as an overlay anchored to particular real-world objects viewed through a see-through display screen. The XR headset 150 may additionally or alternatively be configured to display on the display device 438 video streams from cameras mounted to one or more XR headsets 150 and other cameras.

Electrical components of the XR headset 150 can include a plurality of cameras 430, a microphone 432, a gesture sensor 434, a pose sensor (e.g., inertial measurement unit (IMU)) 436, the display device 438, and a wireless/wired communication interface 440. The cameras 430 of the XR headset 150 may be visible light capturing cameras, near infrared capturing cameras, or a combination of both.

The cameras 430 may be configured to operate as the gesture sensor 434 by tracking for identification user hand gestures performed within the field-of-view of the camera(s) 430. Alternatively, the gesture sensor 434 may be a proximity sensor and/or a touch sensor that senses hand gestures performed proximately to the gesture sensor 434 and/or senses physical contact, e.g., tapping on the sensor 434 or its enclosure. The pose sensor 436, e.g., IMU, may include a multi-axis accelerometer, a tilt sensor, and/or another sensor that can sense rotation and/or acceleration of the XR headset 150 along one or more defined coordinate axes. Some or all of these electrical components may be contained in a head-worn component enclosure or may be contained in another enclosure configured to be worn elsewhere, such as on the hip or shoulder.

As explained above, a surgical system includes the camera tracking system 200 which may be connected to a computer platform 400 for operational processing and which may provide other operational functionality including a navigation controller 404 and/or of an XR headset controller 410. The surgical system may include the surgical robot 100. The navigation controller 404 can be configured to provide visual navigation guidance to an operator for moving and positioning a surgical tool relative to patient anatomical structure based on a surgical plan, e.g., from a surgical planning function, defining where a surgical procedure is to be performed using the surgical tool on the anatomical structure and based on a pose of the anatomical structure determined by the camera tracking system 200. The navigation controller 404 may be further configured to generate navigation information based on a target pose for a surgical tool, a pose of the anatomical structure, and a pose of the surgical tool and/or an end effector of the surgical robot 100. The navigation information may be displayed through the display device 438 of the XR headset 150 and/or another display device to indicate where the surgical tool and/or the end effector of the surgical robot 100 should be moved to perform a surgical procedure according to a defined surgical plan.

The electrical components of the XR headset 150 can be operatively connected to the electrical components of the computer platform 400 through the wired/wireless interface 440. The electrical components of the XR headset 150 may be operatively connected, e.g., through the computer platform 400 or directly connected, to various imaging devices 420, e.g., the C-arm imaging device, the O-arm imaging device, other imaging device, the patient image database, and/or to other medical equipment through the wired/wireless interface 440.

The surgical system may include a XR headset controller 410 that at least partially resides in the XR headset 150, the computer platform 400, and/or another system component connected via wired cables and/or wireless communication links. Various functionality is provided by software executed by the XR headset controller 410. The XR headset controller 410 is configured to receive information from the camera tracking system 200 and the navigation controller 404, and to generate an XR image based on the information for display on the display device 438.

The XR headset controller 410 can be configured to operationally process frames of tracking data from tracking cameras from the cameras 430 (tracking cameras), signals from the microphone 1620, and/or information from the pose sensor 436 and the gesture sensor 434, to generate information for display as XR images on the display device 438 and/or for display on other display devices for user viewing. Thus, the XR headset controller 410 illustrated as a circuit block within the XR headset 150 is to be understood as being operationally connected to other illustrated components of the XR headset 150 but not necessarily residing within a common housing or being otherwise transportable by the user. For example, the XR headset controller 410 may reside within the computer platform 400 which, in turn, may reside within the cabinet 330 of the camera tracking system 200, the cabinet 106 of the surgical robot 100, etc.

### Bone Surface Acquisition Through Palpation ("Painting") Using Ball Tip Stylus:

Various embodiments are directed to registering patient anatomy in an algorithm for computer assisted navigation during surgery through the acquisition of particular landmarks on bones using a process of palpating the surface of the bones with a tracked ball tip stylus. Location of a bone landmark may be acquired and registered by a single touch of the tracked ball tip stylus. A surface of the bone may be acquired and registered by multiple individual touches of, e.g., tapping, the tracked ball tip stylus and/or by touching and then maintaining contact with the surface while moving the ball along the surface, e.g., "painting" the surface while being tracked. Defined landmarks (e.g. most distal and posterior points) can be extracted and other measurements of the bone surface can be performed and concurrently registered for computer assisted navigation. The surface of the bone can be defined (recreated) based on a cloud of points collected by moving the ball of the tracked ball tip stylus on a surface of the bone.

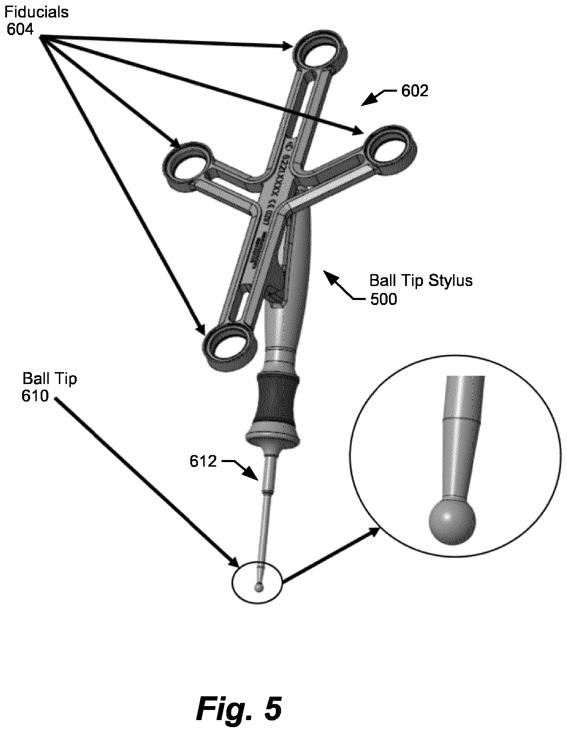

FIG. 5 illustrates the ball tip stylus 500 configured in accordance with some embodiments of the present disclosure. Referring to FIG. 5, the ball tip stylus 500 includes a ball 610 at a tip that is connected through an interconnecting member 612 to a reference element 602 having an array of fiducials 504. The fiducials 504 may be any shape, such as disks, spheres, etc., may be any color, and may be passive to reflect light or active to emit light.

Tip shape can have a considerable influence on how easy and precise it is for a user to move the tip across a bone. It has been determined that a ball (spherical) tip glides more easily and consistently on the bone surface but requires taking into account the radius of the tip when defining a location of the surface. The bigger the ball radius is, the easier the gliding capabilities are, but the more difficult it is to access certain areas of bones of a patient, for example a acetabular cartilage or acetabular fossa of a pelvis.

The user, e.g., surgeon, can manipulate the ball 610 of the ball tip stylus 500 to sweep the surface of the bone or cartilage. Doing so, the camera tracking system 200 can measure the location of the ball 610 in a continuous operation and output a cloud of location points. Alternatively, the user can subsequently measure sufficient amount of points by touching them one-by-one with the ball 610 of the ball tip stylus 500 being tracked by the camera tracking system 200.

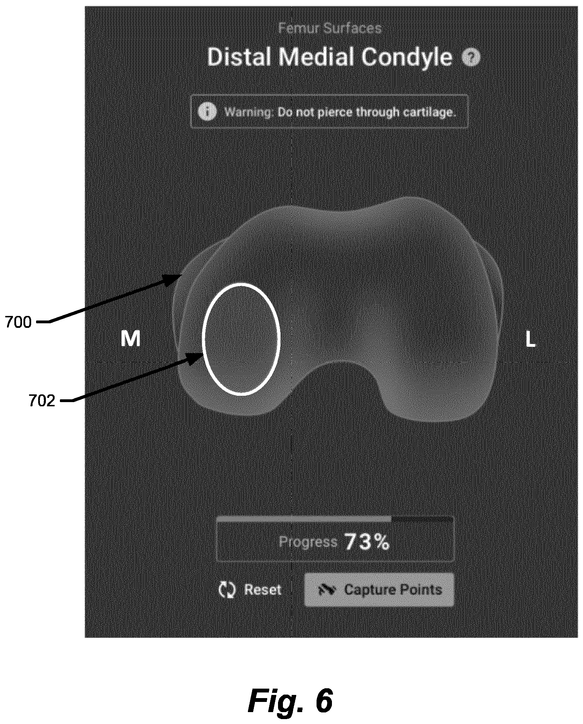

The user may be guided by displayed and/or audible instructions generated by a software application, e.g., algorithm for computer assisted navigation, to acquire a defined surface area of bone(s). FIG. 6 illustrates a user interface that is displayed on a display device to guide a user through registration of condylar surfaces 700 by palpation using the ball tip stylus 500 in accordance with some embodiments of the present disclosure. While condylar surfaces are illustrated with reference to FIG. 6, it should be noted that similar processes can be performed and similar information can be determined/identified with regards to a pelvis of a patient.

The user interface displays an indicia 702 to indicate that the user should use the ball tip stylus 500 to palpate the distal medial condyle to cause the system to define a surface of the distal medial condyle and register the surface in an algorithm for computer assisted navigation during surgery.

The locations (points) acquired by the camera tracking system 200 correspond to the center of the ball 610 during the acquisition by the user. The locations can be used to define an offset-acquired surface of the bone. The offset-acquired surface can then be translated to correspond to the actual surface of the bone based on the radius of the ball 610. The system may use a surface matching algorithm for some of these operations.

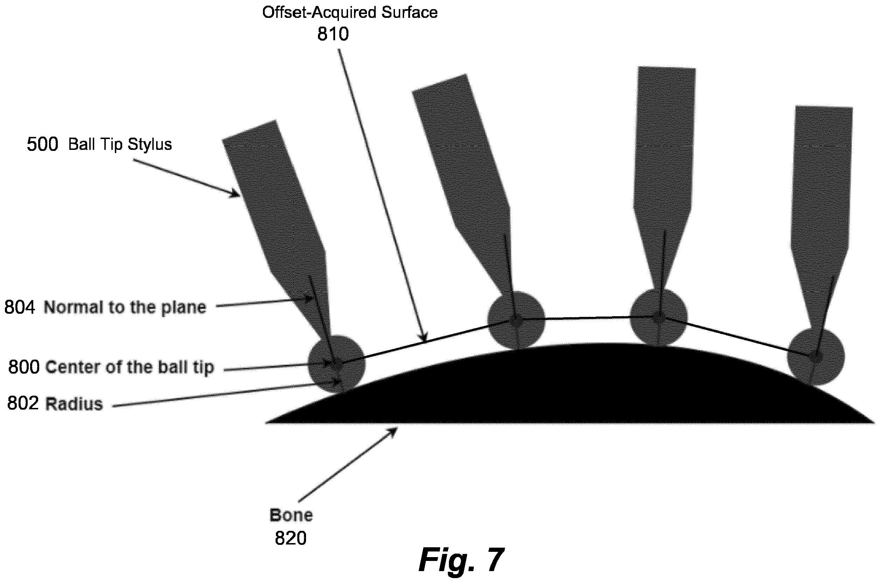

FIG. 7 illustrates a schematic view of operations for defining and then translating an offset-acquired surface 810 of a bone 820 toward the surface of the bone 820 along local normal vectors based on a radius of the ball 610 to define an acquired surface of the bone 820 in accordance with some embodiments of the present disclosure. Referring to FIG. 7, in one illustrative embodiment the operations include to acquire locations of the center 800 of the ball 610 as it is used to palpate a surface of the bone 820. The locations of the center 800 of the ball 610 are then mathematically connected together to define an offset-acquired surface 810 of the bone 820, which is offset from the real bone surface by a distance corresponding to the radius 802 of the ball 610. To match the offset-acquired surface 810 to the real bone surface, the operations determine the local normal vector to the real bone surface for each acquired location of the center 800 of the ball 610. The operations then translate each acquired location of the center 800 of the ball 610 along the normal vector toward the bone 820 by a distance corresponding to the radius 802 of the ball 610. These operations enable the surface to be defined in a manner agnostic to the orientation of the ball tip stylus 500, e.g., when not perpendicular to the palpated surface. The acquired surface of the bone, corresponding to the real bone surface, can then be defined by mathematically connecting together the translated locations.

Landmarks relevant for the surgical procedure (e.g. most posterior condylar points and most distal condylar points for femur; most distal points on the plateaux for tibia; etc.) can be extracted for planning and computer assisted navigation. Based on the acquired surface, a registration algorithm can then match patient anatomy with a 3D model of the bone and/or identify additional landmarks for use in planning and computer assisted navigation.

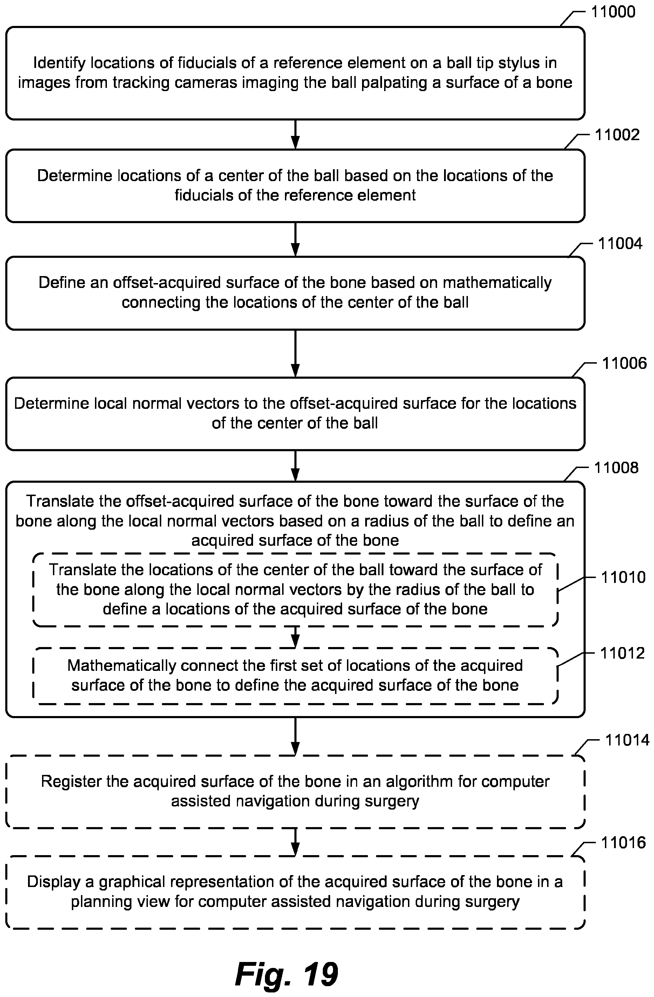

These operations can be more generalized in accordance with some embodiments. FIG. 19 illustrates a flowchart of operation that can be performed by a computer platform of a system for computer assisted navigation during surgery, in accordance with some embodiments of the present disclosure.

Referring to FIG. 19, the operations can include to identify 11000 locations of fiducials 504 of a reference element 602 on a ball tip stylus 500 in images being obtained from tracking cameras 204 with at least partially overlapping field-of-views imaging the ball tip stylus 500 with a ball 610 on the ball tip stylus palpating (contacting) the surface of the bone 820. The operations determine 11002 locations of the center 800 of the ball 610 based on the locations of the fiducials 504 of the reference element 602. The operations define 11004 an offset-acquired surface 810 of the bone based on mathematically connecting the locations of the center 800 of the ball 610. The operations determine 11006 local normal vectors 804 to the offset-acquired surface 810 for the locations of the center 800 of the ball 610. The operations translate 11008 the offset-acquired surface 810 of the bone 820 toward the surface of the bone 820 along the local normal vectors 804 based on the radius 802 of the ball 610 to define an acquired surface of the bone 820, which ideally corresponds precisely to the real surface of the bone 820.

The operation to translate 11008 the offset-acquired surface 810 of the bone 820 toward the surface of the bone 820 along the local normal vectors 804 based on the radius 802 of the ball 610 to define the acquired surface of the bone 820, may include to translate 11010 the locations of the center 800 of the ball 610 toward the surface of the bone 820 along the local normal vectors 804 by the radius 802 of the ball 610 to define a first set of locations of the acquired surface of the bone 820. The operations may then mathematically connect 11012 the locations of the acquired surface of the bone 820 to define the acquired surface of the bone 820.

As will be explained in further detail below, the operations may further include to register 11014 the acquired surface of the bone 820 in an algorithm for computer assisted navigation during surgery, and/or to display 11016 a graphical representation of the acquired surface of the bone 820 in a planning view for computer assisted navigation during surgery.

Some further embodiments are directed to operations for detecting and treating outlier locations which do not correspond to where the ball 610 is palpating (contacting) the bone 820. During measurement of the bone surface, it may be difficult or not needed to maintain contact with the bone surface. However, without operations to detect and treat such outlier locations, these outlier locations may lead to acquisition of numerous non-relevant points, such as while the ball 610 is moved toward the bone 820 to make contact and/or away from the bone 820 for repositioning. In some embodiment, these non-relevant outlier locations are identified using operations that process acquired locations and prevents outlier locations which are space away from the bone surface from being used when defining the offset-acquired surface or acquired surface of the bone. The outlier locations may be identified based on comparison to other locations which are determined. For example, when the locations are connected and result in a plane or a curved pathway, any locations which deviate from the plane or pathway by at least a threshold distance can be flagged as outliers.

Corresponding operations for identifying and treating outliers can include, when defining 11004 the offset-acquired surface of the bone 820 based on mathematically connecting the locations of the center 800 of the ball 610, can include to identify among the locations of the center 800 of the ball 610 an outlier location of the center 800 of the ball 610 where the ball 610 is not palpating the surface of the bone 820. The operations can then define 11004 the offset-acquired surface of the bone based 820 on mathematically connecting the locations of the center 800 of the ball 610 without mathematically connecting the outlier location of the center 800 of the ball 610 where the ball 610 is not palpating the surface of the bone.

The operation to identify among the locations of the center 800 of the ball 610 an outlier location of the center 800 of the ball 610 where the ball 610 is not palpating the surface of the bone 820, may include to identify the outlier location of the center 800 of the ball 610 where the ball 610 is not palpating (contacting) the surface of the bone 820 based on the outlier location of the center 800 of the ball 610 having at least a first threshold distance, in a direction along the local normal vector 804 to the offset-acquired surface 810, from other ones of the locations of the center 800 of the ball 610 which are within a second threshold distance in a direction along the offset-acquired surface 810.

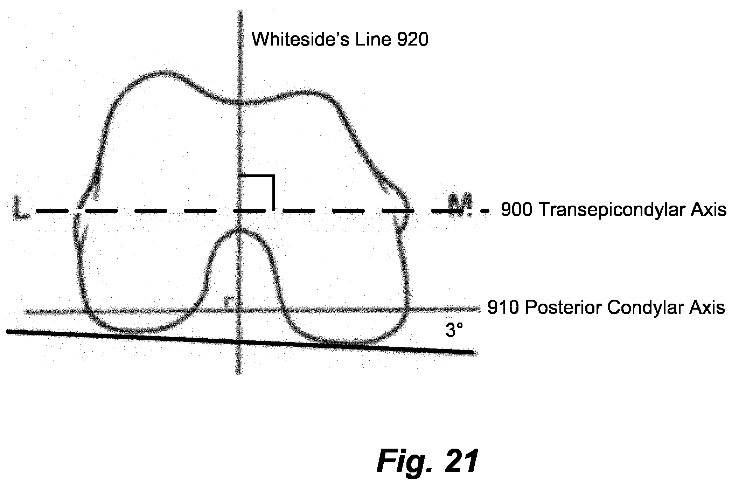

Example operations are now described for acquiring femoral and/or tibial features and axes, these operations can similarly be performed to acquire pelvis features and axes. FIG. 21 illustrates a schematic view of operations for acquiring femoral and/or tibial features in accordance with some embodiments of the present disclosure. While example femoral and/or tibial features are acquired with reference to FIG. 21, similar operations/steps may be performed to acquire/identify other features (e.g., features of a pelvis) of the patient.

Referring to FIG. 21, acquisition of features and other landmarks on the anterior and distal surfaces of the femur as well as tibial plateau through palpation can be performed with the ball tip stylus 500. For example, these areas are naturally exposed, such for the femur, or can be easily accessed, such as by hyperflexion of the joint which provides easy access of about 75% of the tibial plateau. However, the posterior aspect of the condylar surfaces are difficult to acquire through palpation with appropriate level of usability. From a clinical perspective, acquisition of the posterior condylar aspect may be required to determine the posterior condylar axis 910 which is one option to set femoral component internal and/or external rotation during planning.

Operations disclosed here may be used to acquire a posterior aspect of the condylar surfaces using the Whiteside's line 920 and/or the transepicondylar axis 900.

In this operational approach two steps of acquisition is used. In one step, landmarks on the anterior and distal surfaces of femur are acquired and partial registration of the femur is performed. Acquisition of the tibial plateau and full registration of the tibia is performed. Tibial resection is performed. Then, in another step acquisition of the posterior surface of the femoral condyles is performed.

For this operational approach, in the context of FIG. 19 the acquired surface of the bone can define anterior and distal surfaces of a femur palpated by the ball 610. Corresponding operations for one of the steps can include to acquire locations (e.g., 11000-11008 in FIG. 19) of a tibial plateau based on locations of the anterior and distal surfaces of the femur defined by the acquired surface of the bone. The locations of the tibia in a tracking space can be registered (e.g., 11014 in FIG. 19) based on the acquired locations of the tibial plateau. Corresponding operations for the other one of the steps can include to, following tibial resection, the operations identify locations of the fiducials 504 of the reference element 602 on the ball tip stylus 500 in further images obtained from the tracking cameras 204 imaging the ball tip stylus 500 with the ball 610 palpating (contacting) a posterior surface of femoral condyles. The operations determine locations of the center of the ball 610 based on the locations of the fiducials of the reference element, and define an offset-acquired surface of the posterior surface of femoral condyles based on mathematically connecting the locations of the center of the ball 610. The operations determine local normal vectors to the offset-acquired surface of the posterior surface of the femoral condyles for the locations of the center of the ball 610, and translate the offset-acquired surface of the posterior surface of femoral condyles toward the posterior surface of femoral condyles along the local normal vectors based on the radius of the ball 610 to define an acquired surface of the posterior surface of femoral condyles.

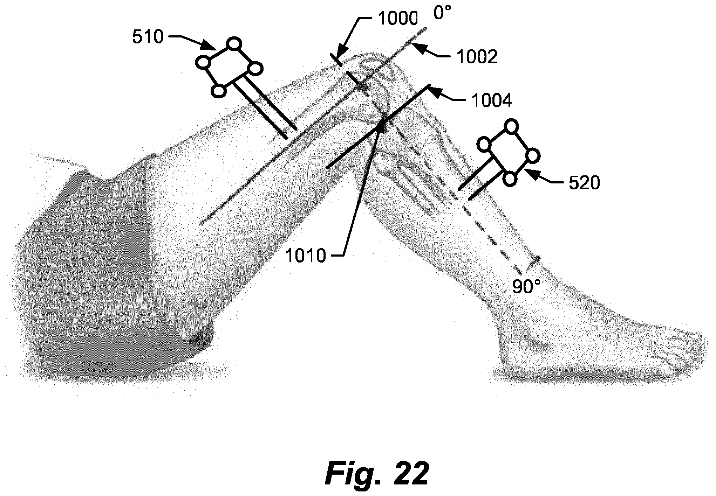

The second operational approach can use the registered tibia with its reference element 520 as a navigated instrument to acquire the posterior condylar axis. FIG. 22 illustrates a schematic view of structure and operations that may be used for the second operational approach for acquiring femoral and/or tibial features. In FIG. 22, a virtual plane 1002 is illustrated as being attached to the femur and determined using landmarks such as a mechanical axis and superior distal aspect of the femur. Another virtual plane 1004 is illustrated as being attached to the tibia and determined from landmarks, such as the mechanical axis and proximal geometry. Another virtual plate 1000 extends perpendicular to the virtual plane 1002 through the tibia to intersect the virtual plane 1004. The intersection of the virtual planes 1000 and 1004 is illustrated as occurring at location 1010, which corresponds to acquisition of the posterior condylar axis using the registered tibia bone.

Corresponding operations can include acquiring locations (e.g., 11000-11008 in FIG. 19) of a tibial plateau based on locations of the anterior and distal surfaces of the femur defined by the acquired surface of the bone. The locations of the tibia in a tracking space can be registered (e.g., 11014 in FIG. 19) based on the acquired locations of the tibial plateau. The operations identify locations of fiducials of the tibial reference element 520 attached to the tibia and locations of fiducials of the femoral reference element 510 attached to the femur in images obtained from the tracking cameras 204 while the ball 610 is palpating the femur. The operations acquire location of the posterior condylar axis based on the registered locations of the tibia and based on the identified locations of the fiducials of the tibial reference element 520 and the femoral reference element 510.

The operation to acquire location of the posterior condylar axis based on the registered locations of the tibia and based on the identified locations of the fiducials of the tibial reference element 520 and the femoral reference element 510, can include to determine a distal femoral plane to be virtually attached to the femur and pass through a posterior surface of femoral condyles. The operations can acquire locations of a tibial plateau based on locations of the anterior and distal surfaces of the femur defined by the acquired surface of the bone and based on the identified locations of the fiducials of the tibial reference element 520. The operations can determine a proximal tibial plane which is virtually attached to the tibia, and determine a posterior condylar axis based on tracking movement of locations of the fiducials of the tibial reference element 520 relative to locations of the fiducials of the femoral reference element 510 as the tibia is rotated relative to the femur.

The operation to determine the posterior condylar axis based on tracking movement of locations of the fiducials of the tibial reference element 520 relative to locations of the fiducials of the femoral reference element 510 as the tibia is rotated relative to the femur, can include to determine the posterior condylar axis based on identifying location of intersection of the distal femoral plane and the proximal tibial plane.

The operation to determine the distal femoral plane can include determining the distal femoral plane to be normal to a femoral mechanical axis, virtually attached to the femur, and pass through the posterior surface of femoral condyles.

The second set of embodiments is now discussed below.

In these embodiments, the systems and embodiments discussed above (along with additional/alternative embodiments discussed below) are used to assist in total hip arthroplasty surgery. For example, the systems and embodiments may be used to prepare the bones (e.g., pelvis acetabulum and femur) and place corresponding implants in the patient.

Certain landmarks and/or planes may be useful in registering or determining the orientation of a patient in 3D space of the operating room. For example, as part of a computer assisted navigation workflow during surgery, the system can register or determine an orientation of an anterior pelvic plane (APP) and/or a functional pelvic plane (FPP) of the patient to determine orientation of the patient on an operating room table during total hip arthroplasty (THA) surgery.

The system may provide computer assisted navigation throughout the entire spectrum of care (pre-operative, intraoperative and post-operative). Accordingly, the system can be configured to perform multiple workflows. Some workflows use scans of the patient (X-ray, Computerized Tomography (CT)) obtained during a pre-operative step.

In some embodiments of the present disclosure, the system performs an imageless workflow in which no pre-operative images are used. Information about the patient anatomy in the operating room (OR) can be obtained by the the surgeon measuring key parameters of the patient's bone using the system. For example, a computer platform of the system operates to identify location of landmarks (e.g., points, axes, and/or surfaces) on the bone and register the locations either concurrently with the identification or thereafter. The locations can be used to define reference plane(s) (e.g., APP and/or FPP) which, in turn, are used to plan implants and navigate the robot and surgical instruments for THA surgical procedures.

In some embodiments, the only pre-operative use case associated with the imageless workflow may be the initial patient assessment. The surgeon may assess the patient mobility and health status with assistance from sensors (e.g., sensors made by Globus Medical which are attached to the leg), physical exercises, and/or clinical surveys to determine if THA is recommended. Gathered data may then be stored and processed by the system before being analyzed by the surgeon to facilitate a final decision. Subsequently, the data may be reused by an application (e.g., surgery planning application by Globus Medical) to establish the most appropriate implant surgical plan.

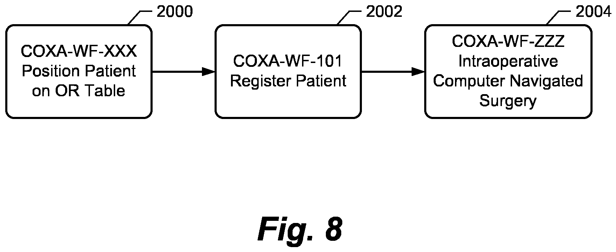

FIG. 8 illustrates a flowchart for an imageless workflow during an intra-operative portion of a THA surgery, in accordance with some embodiments of the present disclosure.

In some embodiments, after positioning the patient on the operating room table (step 2000), some of the operations discussed above and below may be performed during step 2002 to register a patient and before another step 2004 for intraoperative computer navigated surgery. In the case of a hip, a pelvis or acetabulum of the patient is registered in the tracking coordinate system of the camera tracking system 200. As shown in FIG. 1, the pelvis or acetabulum is registered in the optical coordinate system. In one embodiment, the registration is done in an imageless modality without the use of any medical images such as X-rays or CT images from an imaging device.

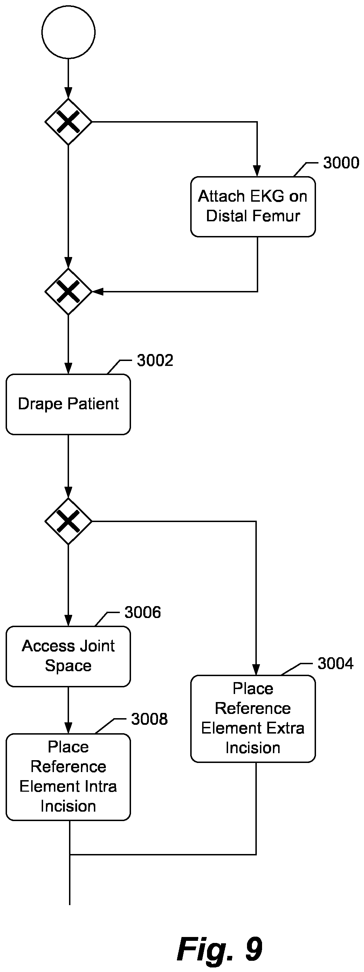

FIG. 9 illustrates a flowchart of a patient preparation process before registration, in accordance with some embodiments of the present disclosure.

The patient preparation process may begin with a patient being positioned in lateral or supine position on the OR table. The patient's body is prepared for registration.

Optionally, in step 3000, an EKG/ECG patch electrode is attacked on a distal end of the patient's femur. The EKG/ECG patch electrode may be placed on the center of the patella or slightly inferior to the center. In some possibly preferrable embodiments, the patch location is in line with the anatomic axis of the femur. This patch may be used to acquire the most distal point of the femur under the drape at a later stage. This patch may be used to track the femur in space (e.g., when the patient's leg is moved during surgery) and may also be used to assist in measuring the patient's leg length. However, in some embodiments, this operation (step 3000) is skipped.

In some embodiments, the EKG/ECG patch electrode includes an adhesive patch that is removably attachable to the patient. In some preferred embodiments, the patch may be black or dark to be more visible to the tracking camera. In other embodiments, the patch electrodes are not visible by the tracking camera as they are under a drape. The patch is not visible by the camera (it is under the patient drape). The patch geometry (like a nipple) will help the surgeon to always touch a single point on the distal part of the femur (anterior patella region) with a navigated stylus/instrument which is trackable by the tracking camera. This ensures that the surgeon always collects the same point to measure the leg length or medio-lateral offset.

In step 3002, the patient body is draped. Then, depending on the surgeon's technique, the navigated pelvis Dynamic Reference Base (DRB) marker array (also referred herein as a reference element) is placed intra-incision (steps 3006-3008) or extra-incision (step 3004) with the help of cortical pins drilled into the pelvic bone. In some embodiments, the DRB is oriented to be visible by the tracking camera(s) (e.g., a stereoscopic tracking camera) installed on the camera tracking system (e.g., camera tracking system 200 of FIG. 1) or the headset (e.g., XR headset 150 of FIG. 1).

In one embodiment, the operation to place the reference element intra-incision, includes using the system to track and navigate access to the joint space (step 3006) and while the reference element is then placed intra-incision.

In an alternative embodiment, the reference element is placed extra-incision (step 3004) and the system does not necessarily need to be used to track and navigate access to the joint space.

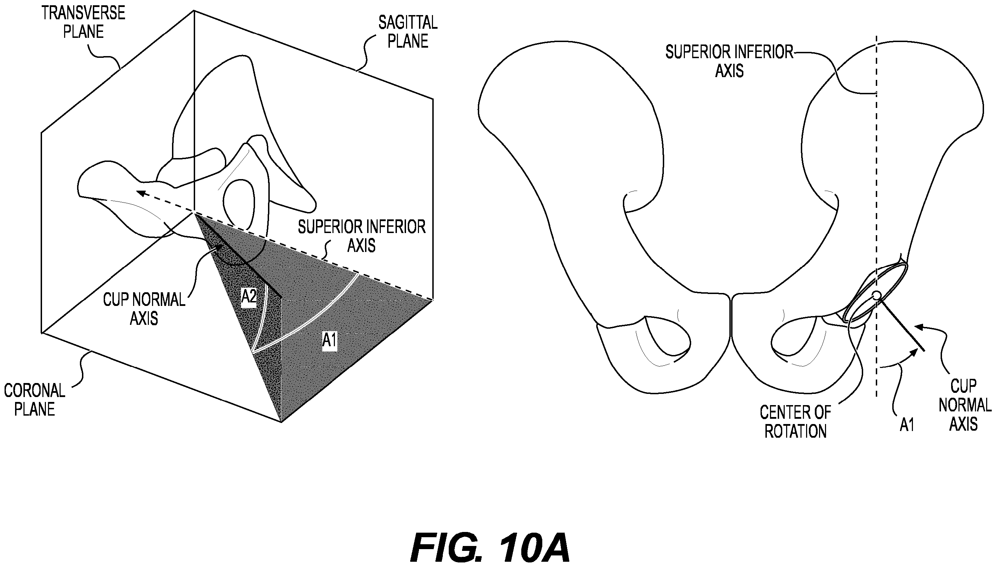

After the reference element has been placed intra-incision or extra-incision, data points and axes can be collected on the patient anatomy with the assistance of navigated instruments and using the pelvis DRB coordinate system as a spatial reference. In addition to this, two pelvic reference planes can be established to plan placement of implants by measuring angular deviations such as inclination and version of the acetabular cup implant as shown in FIGs. 10A-10B.

FIG. 10A illustrates a radiographic inclination angle measured in the coronal plane of the patient, in accordance with some embodiments of the present disclosure. In some embodiments, the surgeon may use a navigated instrument to palpate or paint the surface of the acetabular cavity of the patient to determine a center of rotation of the acetabulum, e.g., as discussed in more detail below.

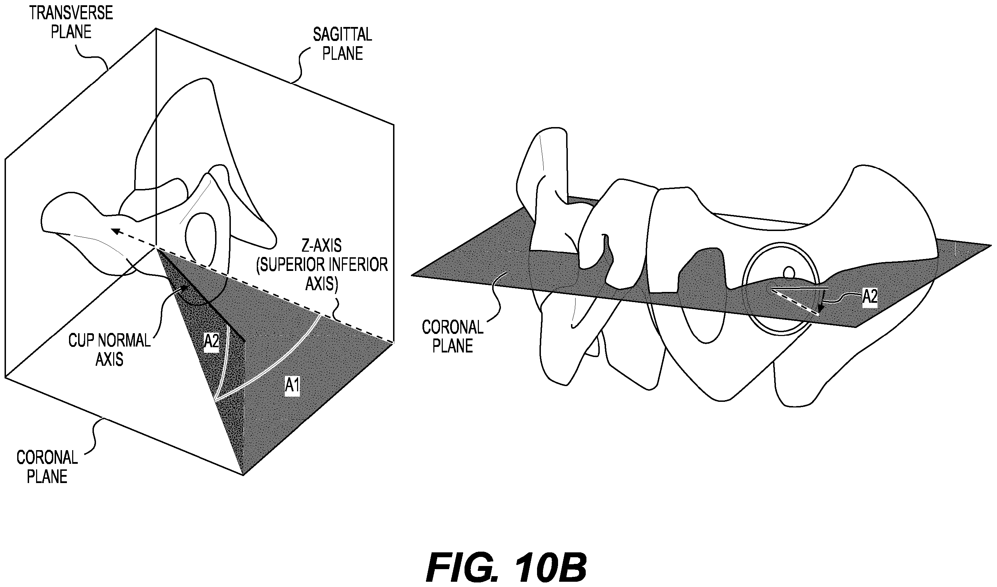

FIG. 10B illustrates a radiographic version angle measured relative to the coronal plane of the patient, in accordance with some embodiments of the present disclosure.

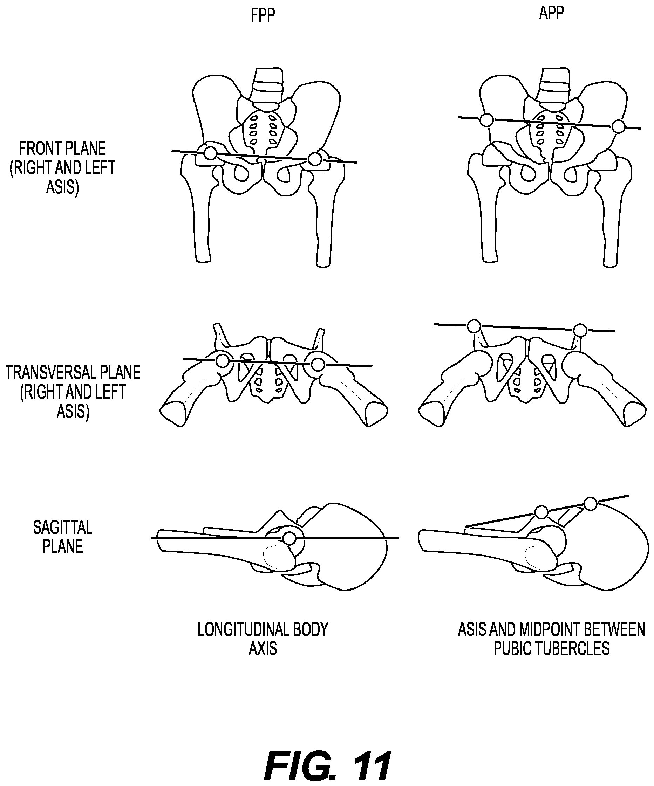

The two pelvic reference planes (or coronal or frontal planes) are called Anterior Pelvic Plane (APP) and Functional Pelvic Plane (FPP). They are determined or defined using different landmarks and axes as shown on FIG. 11 and described in further detail below.

It is to be understood herein that although the user interfaces and associated operations are described as being performed in a certain sequence, they may be performed in other sequences while still being within disclosed embodiments (all the embodiments disclosed in the present disclosure). Moreover, it is not necessary that all of the user interfaces and/or described operations be performed. Instead, fewer operations may be performed while still being within disclosed embodiments.

During a patient registration procedure, landmarks used to register patient anatomy can be extracted using either single point palpation collection or surface painting (resulting in a point cloud of locations).

FIG. 11 illustrates different views of landmarks and axes for registration of a functional pelvic plane (FPP) and an anterior pelvic plane (APP) of a patient, in accordance with some embodiments of the present disclosure.

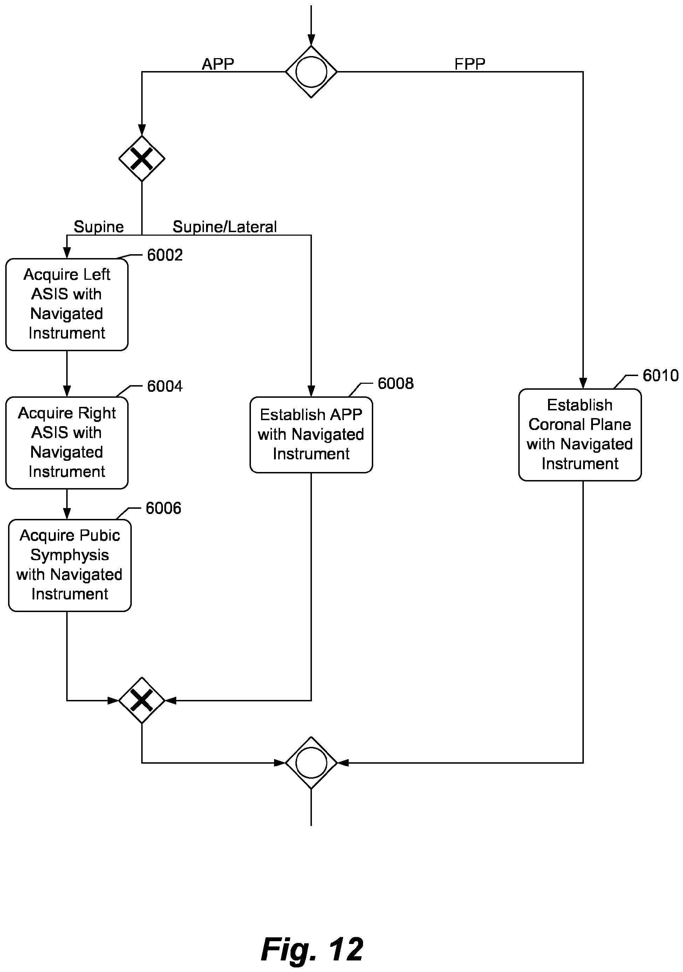

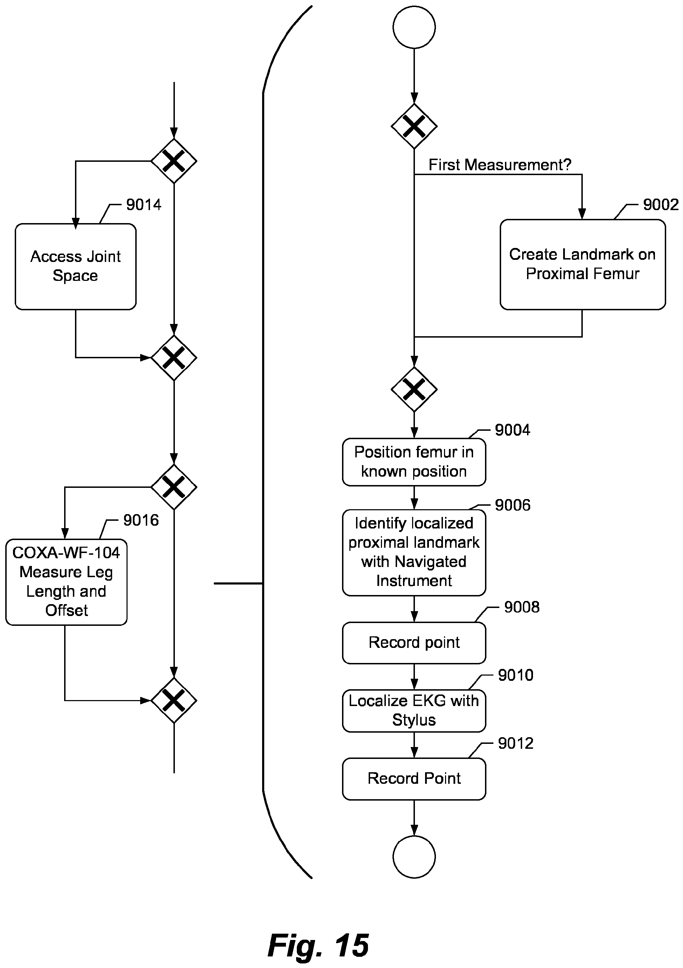

The landmarks and axes used to register the APP and FPP planes are described in more detail with reference to Figures 12 and 15. In some embodiments, the computer platform directs the surgeon to palpate these landmarks for the computer platform to acquire or identify.

FIG. 12 illustrates a flowchart for registration of the APP and FPP of the patient, in accordance with some embodiments of the present disclosure.

For APP plane registration, the accessibility of the landmarks may depend on the selected patient position on the OR table (i.e., supine or lateral position).

If the patient is placed in a supine position on the OR table, a navigated instrument (in this case the stylus) may be used to acquire or identify (step 6002) a left anterior superior iliac spine (ASIS), acquire or identify (step 6004) a right ASIS, and acquire or identify (step 6006) a public symphysis. These landmarks (i.e., the left ASIS, right ASIS, and public symphysis) may then be used to determine an orientation of the APP of the patient.

Alternatively, if the patient is placed in a supine or lateral position then the APP may be established (step 6008) with one of the two navigated instruments discussed in more detail below.

For FPP plane registration, in supine or lateral position, one of the below described navigated instruments may be used to establish (step 6010) the coronal plane. For example, by acquiring or identifying the inferior-superior axes, left-right axes and/or antero-posterior axes of the patient.

Below two navigated instrument operational workflows are described that may be used to establish the APP (step 6008) or establish the coronal plane (step 6010).

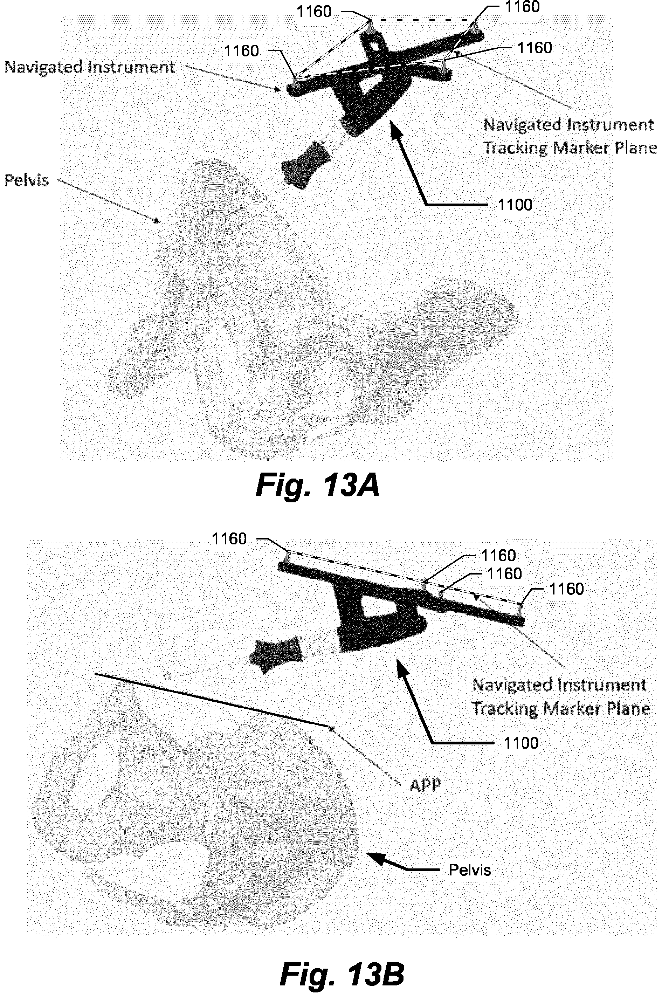

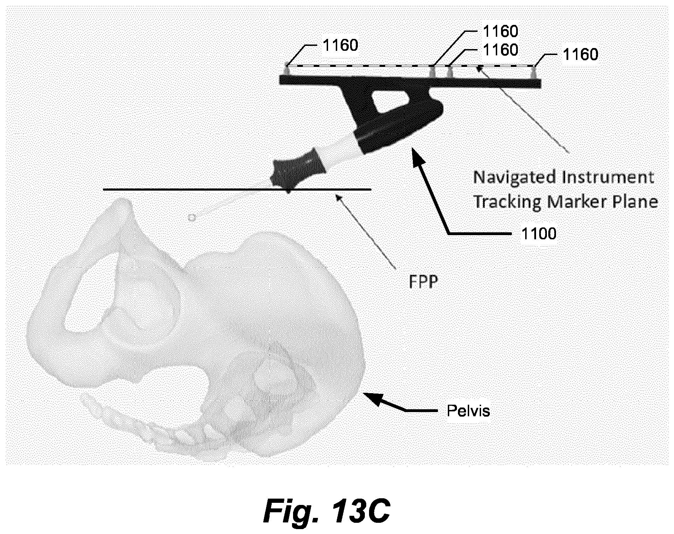

FIGs. 13A-C illustrates examples of the registration (i.e., establishment) of the APP and/or FPP using a tracking marker plane of a navigated instrument, in accordance with some embodiments of the present disclosure.

The first design (shown in FIGs. 13A-C) may be a fully passive navigated instrument 1100. The navigated instrument 1100 may include fiducials 1160 on a reference element of the navigated instrument 1100. These fiducials 1160 may form a navigated instrument tracking marker plane that is the plane that intersects through these fiducials 1160. It is noted that although four fiducials are illustrated in FIGs. 13A-C, any plurality of fiducials may be used to form the navigated instrument tracking marker plane.

As shown in FIGs. 13B-C, the instrument may be positioned by the surgeon such that the plane of the fiducials is parallel to the APP or FPP of the patient's pelvis. Once the plane is parallel to the APP or FPP, the surgeon may trigger capture (e.g., through depression of a foot pedal) by the system of this plane; thereby, establishing the APP and/or FPP with the navigated instrument.

In some embodiments, the tracking marker plane is positioned to be parallel with the APP or FPP while the navigated instrument palpates a landmark of the patient.

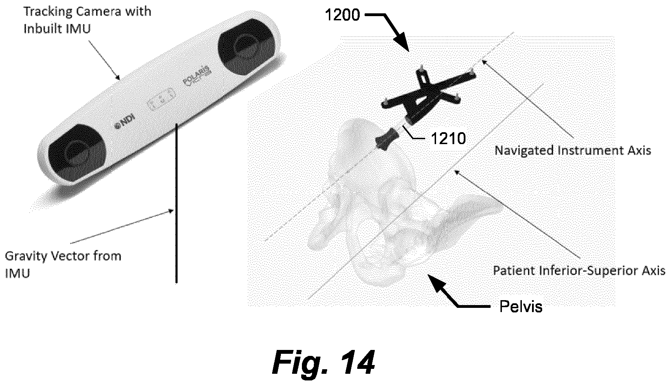

FIG. 14 illustrates examples of the registration of the APP and/or FPP using an axis of the navigated instrument and a tracking camera with an inbuilt inertial measurement unit (IMU), in accordance with some embodiments of the present disclosure.

This second design (shown in FIG. 14) may include: (1) a passive and tracked alignment rod (or bar) 1210 that is attached to or forms a part of the navigated instrument 1200, and the tracked alignment rod 1210 may be used by the surgeon to identify the longitudinal axis (i.e., inferior-superior axis) of the patient by aligning the rod 1210 to be parallel with the patient's inferior-superior axis; and (2) an active inertial measurement unit (IMU) sensor measuring the gravitational force vector that can be used to identify either the antero-posterior axis of the patient in supine position or the left-right axis of the patient in lateral position. This may be determined because when the patient is on their back on the OR table, the gravitational force vector can be normal to the coronal plane. Alternatively, when the patient is on their side, the gravitational force vector can be parallel to the coronal plane.

In some embodiments, the tracked alignment rod may be held parallel to the patient's inferior-superior axis while palpating a landmark of the patient.

Alternatively, the data that is collected from an IMU may be provided by the same navigated instrument 1200 or another device in the system such as another instrument (e.g., a tracked power tool such as a drill, saw-handpiece, etc.), another camera, the XR headset (e.g., XR headset 150 of FIG. 1), etc. that has an IMU sensor.

FIG. 15 illustrates a flowchart of operations for measuring a leg length and offset of the patient, in accordance with some embodiments of the present disclosure.

After APP and/or FPP plane registration, the initial or pre-operative leg length and medio-lateral femur positioning offset may be acquired (step 9016) by accessing (step 9014) the hip joint space and distal part of the femur (see left side of FIG. 15). As shown on the right side of FIG. 15, two points may be acquired or identified to determine the position of the femur with respect to the pelvis.

Optionally, for a first measurement or an measurement before the below-discussed measurements, the system may direct the surgeon to create a landmark on the proximal femur of the patient (step 9002). This landmark may correspond to a surgeon defined landmark such as a divot on the bone anatomy which may allow the computer platform to create a line in 3D space relative to the patch (e.g., the patch placed in step 3000 of FIG. 9) once the patch is also identified (step 9010).

In step 9004, the femur may be positioned in a known position. For example, the system may direct the surgeon to position the femur of the patient in a certain position that is known to the system.

In step 9006, the system may identify a first landmark. The first landmark may be localized in the proximal femoral greater trochanter region, as close as possible to the exit point of the femoral anatomical axis. When identifying the location of the first landmark, the surgeon may need to pay attention to be away from the femoral neck bone portion that will be cut and removed during the procedure. The point in space where the first landmark is identified may be recorded (step 9008) in a memory of the computer platform of the system.

In step 9010, the system may identify a second landmark. The second landmark is identified/acquired on the distal part of the femur by localizing and palpating with the stylus the EKG patch electrode placed on the patient before draping (step 3000 of FIG. 9). This point in space, where the second landmark is identified, may be recorded (step 9012) in the memory of the system.

Using this information (e.g., landmarks identified in steps 9006 and 9010) the system (i.e., computer platform) may be able to determine leg length delta and offset which identifies how far the patient's leg has moved from the patch.

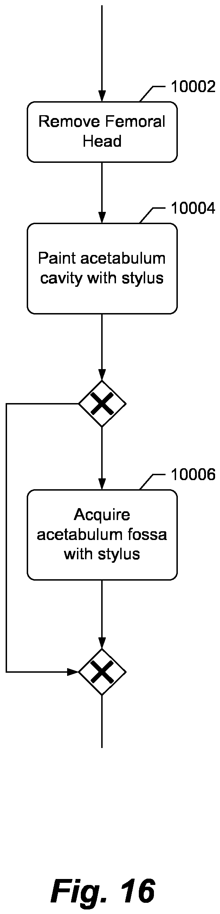

FIG. 16 illustrates a flowchart for registration of a pelvic acetabulum of the patient, in accordance with some embodiments of the present disclosure.

To define the APP and FPP origins, the pelvic acetabular center of rotation is determined by the system directing the surgeon to remove (step 10002) the femoral head of the patient from the acetabular cavity. The acetabular cavity may be made accessible by cutting the femoral neck and by removing the femoral head from the acetabular cavity. In some embodiments, a cork screw instrument may be used to remove the femoral head from the acetabular cavity. The surface of the acetabular cavity can then be painted (step 10004) using the navigated instrument (e.g., the stylus).

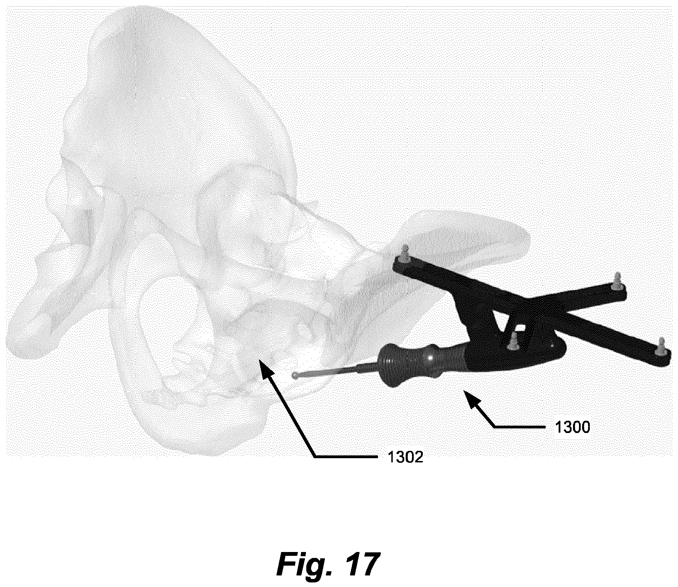

FIG. 17 illustrates operations painting the acetabular cavity 1302 with the navigated instrument 1300, in accordance with some embodiments of the present disclosure. The surgeon uses the navigated instrument 1300 (e.g., stylus) to palpate the surface of the acetabular cavity1302, the tracking camera measures the position of the ball on the end of the stylus in a continuous way and provides a cloud of points for the measured positions (locations). At the same time, the tracking camera may also monitor and track the pose of the patient DPB 116 attached to the pelvis such that the pose of the stylus can be tracked relative to the pose of the patient DRB. Alternatively, the surgeon may subsequently measure a predefined number or percentage of points by palpating them one-by-one. Based on these points and the tracking data of the stylus and patient DRB, the center of rotation of the pelvic acetabular is determined. Additionally, based on these points, the surface of the acetabular cavity may be registered in the system and/or a 3D model may be generated or modified based on the these points.

Next, the acetabular cavity shape can be recreated (e.g., in a 3D model) by the system based on the measured cloud of points and using other algorithms, e.g., for outlier removals and surface fitting.

Optionally, the deepest point in the acetabular fossa may be identified or acquired (step 10006) to provide information on the maximum acetabular reaming depth. For example, the ball of the stylus may be pressed into the acetabular fossa, and the deepest point identified may be acquired or identified by the system as the deepest point in the acetabular fossa. Alternatively, as the acetabular cavity is painted with the navigated instrument, the deepest point in the acetabular fossa may be determined to correspond to the deepest point among the points identified or acquired while painting the acetabular cavity. In some embodiments, step 10006 may be the last step proposed to the surgeon to register the patient with the imageless workflow.

In a similar way to the pre-operative patient assessment, a post-operative patient assessment can be executed by the surgeon to assess the quality of the THA surgery and the benefits to the patient. The workflow can include a patient mobility evaluation, e.g., with data collected from physical exercises and sensor feedback (e.g., attached to the leg), and which can be compared to clinical surveys to confirm the patient's health status. All gathered post-operative information can be automatically compared by the system to the pre-operative information.

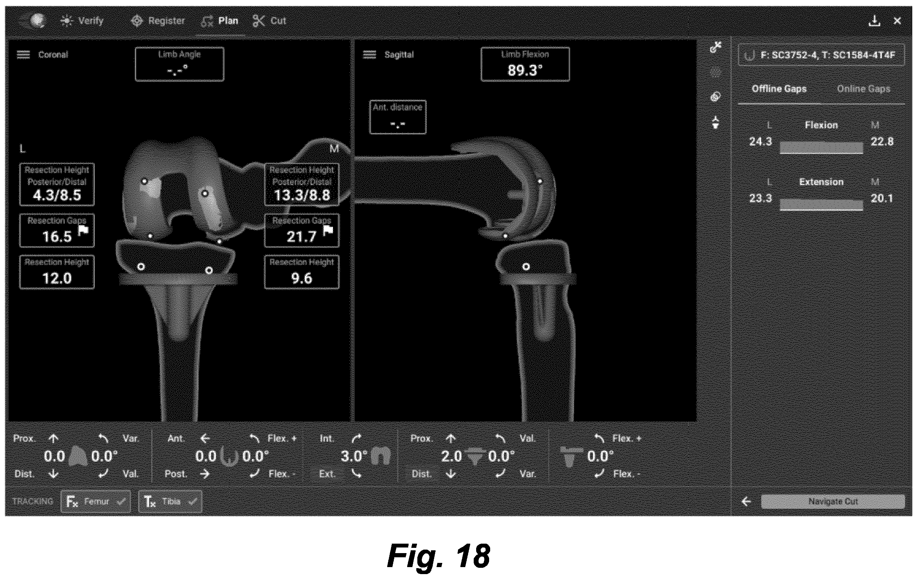

FIG. 18 illustrates a user interface for a planning view which displays a graphical representation of a bone with extracted (e.g., registered) landmarks (shown as bright points), and a graphical representation of what surfaces have been acquired through palpation operations. Although the image represents a knee, similar images may be used to display a pelvis with its acetabulum shown and acquired landmarks.

The bone palpation operations can be used in many ways to acquire and extract landmarks, and which may vary based on whether the operations are for a computer assisted navigation procedure (e.g., robotic) versus manual navigated procedure, and whether the operations are used with an imageless or CT-based procedure.

For example, in a patient registration workflow for computer assisted navigation procedure or manual navigated procedure with imageless or CT-based images, the bone palpation operations can be used to acquire and extract landmarks.

In a workflow for display bone 3D model for computer assisted navigation procedure: for an imageless process, a generic bone model can have dimensions updated based on acquired and extracted landmarks through bone palpation operations; or for CT-based image process a reconstructed 3D model of the patient anatomy can be generated based on pre-operative CT images and based on acquired and extracted landmarks through bone palpation operations.

In a workflow for display bone 3D model for manual navigated procedure: for an imageless process, a generic bone model may not have dimensions updated based on acquired and extracted landmarks through bone palpation operations; or for CT-based image process a reconstructed 3D model of the patient anatomy can be generated based on pre-operative CT images and may or may not be based on acquired and extracted landmarks through bone palpation operations.

In a workflow for display of navigated instruments together with bone model for computer assisted navigation procedure: for imageless process no navigated instrument may be displayed together with the bone 3D model; or for CT-based image process a navigated saw blade may be displayed together with the bone 3D model.

In a workflow for display bone 3D model for manual navigated procedure, for imageless or CT-based image process a navigation guide may be displayed together with a bone 3D model to guide the user for cut block localization pins insertion.

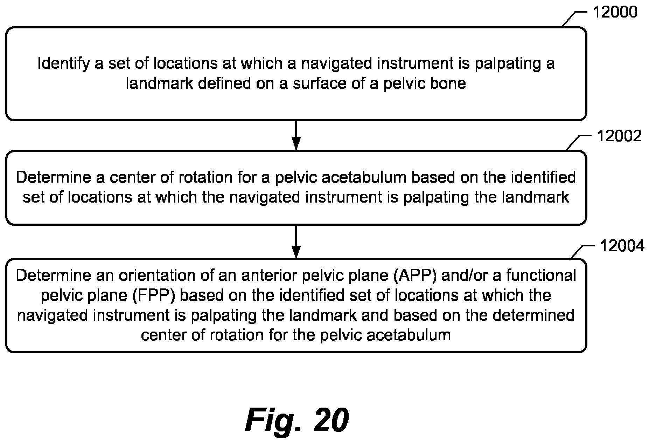

FIG. 20 illustrates a flowchart of additional or alternative operations performed by the computer platform (e.g., computer platform 400 of FIG. 4) of the system for computer assisted navigation during surgery of the patient, in accordance with some embodiments of the present disclosure.

In some embodiments, the computer platform is operative to identify a set of locations at which a navigated instrument (e.g., the stylus of FIG. 5 or the navigated instruments of FIGs. 13A-14) is palpating a landmark defined on a surface of a pelvic bone of a patient. For example, this operation may be performed similar to operations/steps 6002-6010 of FIG. 12, 9006 and 9010 of FIG. 15, and 10004-10006 of FIG. 16.

The computer platform may be further operative to determine a center of rotation for a pelvic acetabulum of the patient based on the identified set of locations at which the navigated instrument is palpating the landmark. For example, the center of rotation for the pelvic acetabulum may be performed similar to at least a portion of the operations/steps performed with regards to FIG. 16.

The computer platform may be further operative to determine an orientation of an anterior pelvic plane (APP) and/or a functional pelvic plane (FPP) of the patient based on the identified set of locations at which the navigated instrument is palpating the landmark and based on the determined center of rotation for the pelvic acetabulum. For example, these operations may be performed similar to the operations of FIG. 12.

In some embodiments, to determine the orientation of the APP, the landmark is defined as the right ASIS, left ASIS, and pubic symphysis of the pelvic bone of the patient. Alternatively, in some embodiments, to determine the orientation of the APP based on the identified set of location at which the navigated instrument is palpating the landmark, the computer platform is further operative to identify an inferior-superior axes, a left-right axes, and/or an antero-posterior axes of the pelvic bone of the patient.

In some embodiments, to determine the orientation of the FPP based on the identified set of locations at which the navigated instrument is palpating the landmark, the computer platform is further operative to identify an inferior-superior axes, a left-right axes, and/or an antero-posterior axes of the pelvic bone of the patient. For example, the identification of the inferior-superior axes, the left-right axes, and/or the antero-posterior axes of the pelvic bone of the patient may be performed similarly as described with reference to FIG. 14. In some embodiments, when determining the center of rotation for the pelvic acetabulum, the landmark is defined as the deepest point of the acetabular fossa of the pelvic bone.

In some embodiments, the inferior-superior axes is identified by a rod on the navigated instrument being parallel to the inferior superior axes while the navigated instrument is palpating the landmark.

In some embodiments, the computer platform is further operative to identify another location at which the navigated instrument is palpating a deepest point on an acetabular fossa. The computer platform may further be operative to determine a maximum acetabular reaming depth based on the identified another location at which the navigated instrument palpated the deepest point on the acetabular fossa.

In some embodiments, the orientation of the APP and/or the FPP, and the center of rotation for pelvic acetabulum, is determined without the use of pre-operative images.

In some embodiments, the computer platform is further operative to register the determined orientation of the APP and/or the FPP in an algorithm for computer assisted navigation during surgery. Additionally, in some embodiments, the computer platform is further operative to display a graphical representation of the determined orientation of the APP and/or the FPP in a planning view for computer assisted navigation during surgery.

In some embodiments, after determining the orientation of the APP and/or the FPP based on the identified set of locations at which the navigated instrument is palpating the landmark and based on the determined center of rotation for the pelvic acetabulum, the computer platform is further operative to identify a first location, separate from the set of locations, at which the navigated instrument palpates a proximal femoral greater trochanter region at least proximate to an exit point of a femoral anatomical axis. In these embodiments, the computer platform is further operative to identify a second location, separate from the set of locations, at which the navigated instrument palpates a distal part of the femur where an electrocardiogram (EKG) patch electrode is located on the patient. Additionally, in some embodiments, the computer platform is further operative to determine length of a leg of the patient and a medio-lateral femur positioning offset based on the identified first location and identified second location.

Additionally, in these embodiments, the identification of the another location, separate from the set of locations, at which the navigated instrument palpates the proximal femoral greater trochanter region at least proximate to the exit point of the femoral anatomical axis is performed after a femur of the patient is placed in a known position.

In some embodiments, the navigated instrument comprises a ball tip stylus. For example, the ball tip stylus may correspond to the ball tip stylus of FIG. 5. The computer platform can be operative to identify the set of locations at which the navigated instrument is palpating the landmark by identifying locations of fiducials of a reference element on a ball tip stylus in images being obtained from tracking cameras with at least partially overlapping field-of-views imaging the ball tip stylus with a ball on the ball tip stylus palpating the surface of the bone. Further, the computer platform may be operative to: identify the set of location by determining locations of a center of the ball based on the locations of the fiducials of the reference element; define an offset-acquired surface of the bone based on mathematically connecting the locations of the center of the ball; determine local normal vectors to the offset-acquired surface for the locations of the center of the ball; and translate the offset-acquired surface of the bone toward the surface of the bone along the local normal vectors based on a radius of the ball to define an acquired surface of the bone.

In some embodiments, the computer platform is further operative to determine a center of rotation for a pelvic acetabular of the patient based on the identified set of locations at which the navigated instrument is palpating the landmark comprising an inside of an acetabulum cavity of the patient, and determine the origin of the APP and/or FPP based on the center of rotation for the pelvic acetabular of the patient. In these embodiments, the determination of the center of rotation for the pelvic acetabular of the patient can be performed after a femoral head of a femur of the patient is removed from the acetabular cavity. Additionally or alternatively, the computer platform is further operative to generate a model of the shape of the acetabulum cavity based on the identified set of locations at which the navigated instrument is palpating the landmark comprising the inside of the acetabulum cavity of the patient.

In some embodiments, the computer platform is further operative to register in an algorithm for computer assisted navigation during surgery, at least one of the determined orientation of the APP, determined orientation of the FPP, and determined center of rotation for the pelvic acetabulum.

In some embodiments, the orientation of the APP and/or the FPP of the patient is determined further based on a gravity vector of an inbuilt inertial measurement unit (IMU) of a tracking camera of the system.

As described above and in summary, there may be at least two different methods of imagelessly registering the pelvis to the tracking coordinate of the tracking system (e.g., optical coordinate system). In both methods, a center of rotation of the acetabulum is determined, for example, based on palpation either by touching multiple points on the inside of the acetabulum a with a navigated instrument/probe 1100 or by continuously surface painting without lifting the navigated instrument/probe 1100. In a first method, an FPP is derived by a physician lining up a plane or axis defined by the navigated instrument along or parallel to the FPP. Once the center of rotation and FPP are determined, the system (either a navigation system or a combined navigation and robot system 100) has sufficient information to register the acetabulum in the coordinate system (e.g., optical coordinate system) of the camera tracking system 200. Registration may allow a navigation system or robotic system to track any navigated instrument or end effector or any tool attached to the end effector relative to the pelvis as tracked by a patient dynamic reference base 116 attached to the pelvis.

In a second embodiment, an APP is derived by either touching various known points (e.g., left and right ASIS and pubic symphysis) with a navigated instrument, or by a physician lining up a plane or axis defined by the navigated instrument along or parallel to the APP. With the center of rotation and APP determined, the system (either a navigation system or a combined navigation and robot system 100) has sufficient information to register the acetabulum in the coordinate system (e.g., optical coordinate system) of the camera tracking system 200.

In both of the methods described above, the tracking system may be constantly monitoring and tracking the pose of the patient DPB 116 attached to the pelvis while also tracking the navigated instrument 1100 such that the pose of the instrument can be tracked relative to the pose of the patient DRB, at least for purposes of registering the pelvis relative to the patient DPB 116 in the tracking coordinate system of the camera tracking system 200.

### Further Definitions and Embodiments:

In the above-description of various embodiments of present inventive concepts, it is to be understood that the terminology used herein is for the purpose of describing particular embodiments only and is not intended to be limiting of present inventive concepts. Unless otherwise defined, all terms (including technical and scientific terms) used herein have the same meaning as commonly understood by one of ordinary skill in the art to which present inventive concepts belongs. It will be further understood that terms, such as those defined in commonly used dictionaries, should be interpreted as having a meaning that is consistent with their meaning in the context of this specification and the relevant art and will not be interpreted in an idealized or overly formal sense expressly so defined herein.

When an element is referred to as being "connected", "coupled", "responsive", or variants thereof to another element, it can be directly connected, coupled, or responsive to the other element or intervening elements may be present. In contrast, when an element is referred to as being "directly connected", "directly coupled", "directly responsive", or variants thereof to another element, there are no intervening elements present. Like numbers refer to like elements throughout. Furthermore, "coupled", "connected", "responsive", or variants thereof as used herein may include wirelessly coupled, connected, or responsive. As used herein, the singular forms "a", "an" and "the" are intended to include the plural forms as well, unless the context clearly indicates otherwise. Well-known functions or constructions may not be described in detail for brevity and/or clarity. The term "and/or" includes any and all combinations of one or more of the associated listed items.

It will be understood that although the terms first, second, third, etc. may be used herein to describe various elements/operations, these elements/operations should not be limited by these terms. These terms are only used to distinguish one element/operation from another element/operation. Thus, a first element/operation in some embodiments could be termed a second element/operation in other embodiments without departing from the teachings of present inventive concepts. The same reference numerals or the same reference designators denote the same or similar elements throughout the specification.

As used herein, the terms "comprise", "comprising", "comprises", "include", "including", "includes", "have", "has", "having", or variants thereof are open-ended, and include one or more stated features, integers, elements, steps, components or functions but does not preclude the presence or addition of one or more other features, integers, elements, steps, components, functions or groups thereof. Furthermore, as used herein, the common abbreviation "e.g.", which derives from the Latin phrase "exempli gratia," may be used to introduce or specify a general example or examples of a previously mentioned item, and is not intended to be limiting of such item. The common abbreviation "i.e.", which derives from the Latin phrase "id est," may be used to specify a particular item from a more general recitation.

Example embodiments are described herein with reference to block diagrams and/or flowchart illustrations of computer-implemented methods, apparatus (systems and/or devices) and/or computer program products. It is understood that a block of the block diagrams and/or flowchart illustrations, and combinations of blocks in the block diagrams and/or flowchart illustrations, can be implemented by computer program instructions that are performed by one or more computer circuits. These computer program instructions may be provided to a processor circuit of a general purpose computer circuit, special purpose computer circuit, and/or other programmable data processing circuit to produce a machine, such that the instructions, which execute via the processor of the computer and/or other programmable data processing apparatus, transform and control transistors, values stored in memory locations, and other hardware components within such circuitry to implement the functions/acts specified in the block diagrams and/or flowchart block or blocks, and thereby create means (functionality) and/or structure for implementing the functions/acts specified in the block diagrams and/or flowchart block(s).

These computer program instructions may also be stored in a tangible computer-readable medium that can direct a computer or other programmable data processing apparatus to function in a particular manner, such that the instructions stored in the computer-readable medium produce an article of manufacture including instructions which implement the functions/acts specified in the block diagrams and/or flowchart block or blocks. Accordingly, embodiments of present inventive concepts may be embodied in hardware and/or in software (including firmware, resident software, micro-code, etc.) that runs on a processor such as a digital signal processor, which may collectively be referred to as "circuitry," "a module" or variants thereof.

It should also be noted that in some alternate implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. Moreover, the functionality of a given block of the flowcharts and/or block diagrams may be separated into multiple blocks and/or the functionality of two or more blocks of the flowcharts and/or block diagrams may be at least partially integrated. Finally, other blocks may be added/inserted between the blocks that are illustrated, and/or blocks/operations may be omitted without departing from the scope of inventive concepts. Moreover, although some of the diagrams include arrows on communication paths to show a primary direction of communication, it is to be understood that communication may occur in the opposite direction to the depicted arrows.

Many variations and modifications can be made to the embodiments without substantially departing from the principles of the present inventive concepts. All such variations and modifications are intended to be included herein within the scope of present inventive concepts. Accordingly, the above disclosed subject matter is to be considered illustrative, and not restrictive, and the appended examples of embodiments are intended to cover all such modifications, enhancements, and other embodiments, which fall within the spirit and scope of present inventive concepts. Thus, to the maximum extent allowed by law, the scope of present inventive concepts are to be determined by the broadest permissible interpretation of the present disclosure including the following examples of embodiments and their equivalents, and shall not be restricted or limited by the foregoing detailed description.

The invention could be defined, inter alia, by the following examples:
1. A system for computer assisted navigation during surgery, comprising a computer platform operative to:
   identify a set of locations on a surface of a pelvic bone of a patient at which a navigated instrument is palpitating while the navigated instrument is being tracked by a tracking system;
   determine a center of rotation for a pelvic acetabulum of the patient based on the identified set of locations;
   determine an orientation of an anterior pelvic plane (APP) and/or a functional pelvic plane (FPP) of the patient using the navigated instrument while the tracking system is tracking the navigated instrument;
   register the pelvis in a coordinate system of the tracking system based on the determined APP or FPP and the determined center of rotation for the pelvic acetabulum.
2. The system of example 1, wherein to determine the orientation of the APP, the computer platform records locations of the right anterior superior iliac spine, left anterior superior iliac spine, and pubic symphysis of the pelvic bone of the patient based on tracking of the navigated instrument by the tracking system.
3. The system of example 1, wherein to determine the orientation of the FPP, the computer platform is further operative to:
   identify an inferior-superior axes, a left-right axes, and/or an antero-posterior axes of the pelvic bone of the patient based on tracking of the navigated instrument by the tracking system.
4. The system of example 3, wherein the computer platform identifies the inferior-superior axes by recording the pose of the navigated instrument when a rod on the navigated instrument is parallel to the inferior superior axes.
5. The system of example 1, wherein to determine the orientation of the APP, the computer platform is further operative to:
   record the pose of the navigated instrument when the navigated instrument is identifying an inferior-superior axes, a left-right axes, and/or an antero-posterior axes of the pelvic bone of the patient.
6. The system of example 1, wherein when identifying a set of locations on a surface of a pelvic bone of a patient, the computer platform is operative to continuously track a pose of the navigated instrument and a patient dynamic reference base attached to the pelvis.
7. The system of example 1, wherein the computer platform is further operative to:
   identify another location at which the navigated instrument is contacting a deepest point on an acetabular fossa; and
   determine a maximum acetabular reaming depth based on the identified another location.
8. The system of example 1, wherein the orientation of the APP and/or the FPP, and the center of rotation for pelvic acetabulum, is determined without the use of pre-operative images.
9. The system of example 1, wherein the computer platform is further operative to:
   display a graphical representation of the determined orientation of the APP and/or the FPP in a planning view for computer assisted navigation during surgery.
10. The system of example 1, the computer platform is further operative to:
   identify a first location, separate from the set of locations, at which the navigated instrument palpitates a proximal femoral greater trochanter region at least proximate to an exit point of a femoral anatomical axis;
   identify a second location, separate from the set of locations, at which the navigated instrument palpitates a known location of a distal part of the femur; and
   determine a length of a leg of the patient and a medio-lateral femur positioning offset based on the identified first location and identified second location.
11. The system of example 10, wherein the computer platform identifies the first location after a femur of the patient is placed in a known position to confirm a planned leg length.
12. The system of example 1, wherein the navigated instrument comprises a ball tip stylus.
13. The system of example 12, wherein the computer platform is operative to identify the set of locations by:
   identifying locations of fiducials of the ball tip stylus in images being obtained from cameras of the tracking system;
   determining locations of a center of the ball based on the locations of the fiducials of the ball tip stylus;
   defining an offset-acquired surface of the bone based on mathematically connecting the locations of the center of the ball;
   determining local normal vectors to the offset-acquired surface for the locations of the center of the ball; and
   translating the offset-acquired surface of the bone toward the surface of the bone along the local normal vectors based on a radius of the ball to define an acquired surface of the bone.
14. The system of example 13, wherein the computer platform is operative to identify the set of locations by simultaneously tracking the fiducials of the ball tip stylus and a patient dynamic reference base attached to the pelvis.
15. The system of example 1, wherein the computer platform determines the center of rotation for the pelvic acetabulum after a femoral head of a femur of the patient is removed from the acetabular cavity.
16. The system of example 1, where the computer platform is further operative to:
   generate a model of the shape of the acetabulum cavity based on the identified set of locations.
17. The system of example 1, wherein the computer platform is further operative to:
   register in an algorithm for computer assisted navigation during surgery, at least one of the determined orientation of the APP, determined orientation of the FPP, and determined center of rotation for the pelvic acetabulum.
18. The system of example 1, wherein the computer platform is further operative to determine the orientation of the APP or the FPP based on a gravity vector of an inbuilt inertial measurement unit (IMU) of the tracking system.
19. A computer program product comprising a non-transitory computer readable medium storing instructions executable by at least one processor to perform operations for computer assisted navigation during surgery to:
   identify a set of locations on a surface of a pelvic bone of a patient at which a navigated instrument is palpitating while the navigated instrument is being tracked by a tracking system;
   determine a center of rotation for a pelvic acetabulum of the patient based on the identified set of locations;
   determine an orientation of an anterior pelvic plane (APP) and/or a functional pelvic plane (FPP) of the patient using the navigated instrument while the tracking system is tracking the navigated instrument;
   register the pelvis in a coordinate system of the tracking system based on the determined APP or FPP and the determined center of rotation for the pelvic acetabulum.
20. The computer program product of example 19, wherein the computer readable medium stores instructions for the processor to determine the orientation of the FPP by identifying the pose of the navigated instrument by the tracking system while the navigated instrument is positioned along a line parallel to the FPP.

## Claims

1. A system for computer assisted navigation during surgery, comprising a computer platform operative to:
identify a set of locations on a surface of a pelvic bone of a patient at which a navigated instrument is palpitating while the navigated instrument is being tracked by a tracking system;
determine a center of rotation for a pelvic acetabulum of the patient based on the identified set of locations;
determine an orientation of an anterior pelvic plane (APP) and/or a functional pelvic plane (FPP) of the patient using the navigated instrument while the tracking system is tracking the navigated instrument;
register the pelvis in a coordinate system of the tracking system based on the determined APP or FPP and the determined center of rotation for the pelvic acetabulum.

2. The system of Claim 1, wherein to determine the orientation of the APP, the computer platform records locations of the right anterior superior iliac spine, left anterior superior iliac spine, and pubic symphysis of the pelvic bone of the patient based on tracking of the navigated instrument by the tracking system.

3. The system of Claim 1 or 2, wherein to determine the orientation of the FPP, the computer platform is further operative to:
identify an inferior-superior axes, a left-right axes, and/or an antero-posterior axes of the pelvic bone of the patient based on tracking of the navigated instrument by the tracking system.

4. The system of Claim 3, wherein the computer platform identifies the inferior-superior axes by recording the pose of the navigated instrument when a rod on the navigated instrument is parallel to the inferior superior axes.

5. The system of any one of the preceding claims, wherein to determine the orientation of the APP, the computer platform is further operative to:
record the pose of the navigated instrument when the navigated instrument is identifying an inferior-superior axes, a left-right axes, and/or an antero-posterior axes of the pelvic bone of the patient.

6. The system of any one of the preceding claims, wherein when identifying a set of locations on a surface of a pelvic bone of a patient, the computer platform is operative to continuously track a pose of the navigated instrument and a patient dynamic reference base attached to the pelvis.

7. The system of any one of the preceding claims, wherein the computer platform is further operative to:
identify another location at which the navigated instrument is contacting a deepest point on an acetabular fossa; and
determine a maximum acetabular reaming depth based on the identified another location.

8. The system of any one of the preceding claims, wherein the orientation of the APP and/or the FPP, and the center of rotation for pelvic acetabulum, is determined without the use of pre-operative images.

9. The system of any one of the preceding claims, wherein the computer platform is further operative to:
display a graphical representation of the determined orientation of the APP and/or the FPP in a planning view for computer assisted navigation during surgery.

10. The system of any one of the preceding claims, the computer platform is further operative to:
identify a first location, separate from the set of locations, at which the navigated instrument palpitates a proximal femoral greater trochanter region at least proximate to an exit point of a femoral anatomical axis;
identify a second location, separate from the set of locations, at which the navigated instrument palpitates a known location of a distal part of the femur; and
determine a length of a leg of the patient and a medio-lateral femur positioning offset based on the identified first location and identified second location.

11. The system of Claim 10, wherein the computer platform identifies the first location after a femur of the patient is placed in a known position to confirm a planned leg length.

12. The system of any one of the preceding claims, wherein the navigated instrument comprises a ball tip stylus.

13. The system of Claim 12, wherein the computer platform is operative to identify the set of locations by:
identifying locations of fiducials of the ball tip stylus in images being obtained from cameras of the tracking system;
determining locations of a center of the ball based on the locations of the fiducials of the ball tip stylus;
defining an offset-acquired surface of the bone based on mathematically connecting the locations of the center of the ball;
determining local normal vectors to the offset-acquired surface for the locations of the center of the ball; and
translating the offset-acquired surface of the bone toward the surface of the bone along the local normal vectors based on a radius of the ball to define an acquired surface of the bone.

14. The system of Claim 13, wherein the computer platform is operative to identify the set of locations by simultaneously tracking the fiducials of the ball tip stylus and a patient dynamic reference base attached to the pelvis.

15. The system of any one of the preceding claims, wherein the computer platform determines the center of rotation for the pelvic acetabulum after a femoral head of a femur of the patient is removed from the acetabular cavity.
